# EUROPEAN PATENT SPECIFICATION

(11) **EP 1 893 142 B1**
(45) Date of publication and mention of the grant of the patent: **24.04.2013**
(21) Application number: 06770080.7
(22) Date of filing: 08.05.2006
(51) Int. Cl.: A61F 5/00

(54) **INTRAGASTRIC DEVICE FOR TREATING OBESITY**
MAGENSONDE ZUR BEHANDLUNG VON ADIPOSITAS
DISPOSITIF INTRAGASTRIQUE POUR TRAITER L'OBESITE

(30) Priority: 09.05.2005 US 679135 P
(43) Date of publication of application: 05.03.2008
(73) Proprietor: Cook Medical Technologies LLC, Bloomington, IN 47404 (US)
(72) Inventor: HASHIBA, Kiyoshi, CEP-0547-000 Sao Paulo (BR); JONES, Brian, K., Winston-salem, North Carolina 27106 (US); KENNEDY, Kenneth, C., II, Clemmons, North Carolina 27102 (US); SURTI, Vihar, C., Winston-salem, North Carolina 27106 (US)
(74) Representative: Mathys & Squire LLP
(86) International application number: PCT/US2006/017704
(87) International publication number: WO 2006/122019

(56) References cited:
- WO-A-02/091961

## Description

### RELATED APPLICATIONS

This application claims priority to provisional application no. 60/679,135 filed on May 9,2005.

### TECHNICAL FIELD

This invention relates to medical devices, and more particularly to obesity treatment devices that can be placed in the stomach of a patient to reduce the size of the stomach reservoir.

### BACKGROUND OF THE INVENTION

It is well known that obesity is a very difficult condition to treat. Methods of treatment are varied, and include drugs, behavior therapy, and physical exercise, or often a combinational approach involving two or more of these methods. Unfortunately, results are seldom long term, with many patients eventually returning to their original weight over time. For that reason, obesity, particularly morbid obesity, is often considered an incurable condition. More invasive approaches have been available which have yielded good results in many patients. These include surgical options such as bypass operations or gastroplasty. However, these procedures carry high risks, and are therefore not appropriate for most patients.

In the early 1980s, physicians began to experiment with the placement of intragastric balloons to reduce the size of the stomach reservoir, and consequently its capacity for food. Once deployed in the stomach, the balloon helps to trigger a sensation of fullness and a decreased feeling of hunger. These balloons are typically cylindrical or pear-shaped, generally range in size from 200-500 ml or more, are made of an elastomer such as silicone, polyurethane, or latex, and are filled with air, water, or saline. While some studies demonstrated modest weight loss, the effects of these balloons often diminished after three or four weeks, possibly due to the gradual distension of the stomach or the fact that the body adjusted to the presence of the balloon. Other balloons include a tube exiting the nasal passage that allows the balloon to be periodically deflated and re-insufflated to better simulate normal food intake. However, the disadvantages of having an inflation tube exiting the nose are obvious.

The experience with balloons as a method of treating obesity has provided uncertain results, and has been frequently disappointing. Some trials failed to show significant weight loss over a placebo, or were ineffective unless the balloon placement procedure was combined with a low-calorie diet. Complications have also been observed, such as gastric ulcers, especially with use of fluid-filled balloons, and small bowel obstructions caused by deflated balloons. In addition, there have been documented instances of the balloon blocking off or lodging in the opening to the duodenum, wherein the balloon may act like a ball valve to prevent the stomach contents from emptying into the intestines.

Reference is directed to WO 02/091961 in which there is provided an apparatus comprising an intragastric member re-configurable from a first configuration to a second configuration, the first configuration being sufficiently small to permit introduction of said intragastric member into a gastric lumen of a mammal, the second configuration being sufficiently large to prevent said intragastric device from passing through the mammal's pylorus, wherein said intragastric member comprises a plurality of spaced apart openings. The intragastric member comprises an elongate member having a proximal end and a distal end, wherein the elongate member is threaded through the openings of the intragastric member, and the intragastric member is disposed between the proximal end and the distal end of the elongate member.; The apparatus also comprises a proximal stopper engaged to the distal end of the elongate member and a proximal stopper engaged to the proximal end of the elongate member for securing the intragastric member upon delivery into the gastric lumen of the patient. The elongate member can also comprise a cinching member to facilitate delivery of the intragastric member into the gastric lumen of the patient.

Unrelated to the above-discussed methods for treating obesity, it has been observed that the ingestion of certain indigestible matter, such as fibers, hair, fuzzy materials, etc., can collect in the stomach over time, and eventually form a mass called a bezoar. In some patients, particularly children and the mentally handicapped, bezoars often result from the ingestion of plastic or synthetic materials. In many cases, bezoars can cause indigestion, stomach upset, or vomiting, especially if allowed to grow sufficiently large. It has also been documented that certain individuals having bezoars are subject to weight loss, presumably due to the decrease in the size of the stomach reservoir. Although bezoars may be removed endoscopically, especially in conjunction with a device known as a bezotome or bezotriptor, they, particularly larger ones, often require surgery.

What is needed is an intragastric member that provides the potential weight loss benefits of a bezoar or intragastric balloon without the associated complications. Ideally, such a-device should be well-tolerated by the patient, effective over a long period of time, sizable for individual anatomies, and easy to place and retrieve.

### SUMMARY OF THE INVENTION

The foregoing problems are solved and a technical advance is achieved by an illustrative obesity treatment apparatus comprising at least one intragastric member or artificial bezoar made of a digestive-resistant or substantially indigestible material that is introduced into a gastric lumen of a mammal in a first configuration. The intragastric member or artificial bezoar is typically inserted into the gastric lumen in a partially compacted configuration, whereby it is then manipulated into, or allowed to assume, a second expanded configuration sufficiently large to remain within the reservoir of the stomach during normal activities and not be passed through the pylorus and into the intestines. In animals, the present invention has been found to be effective in achieving weight loss over a several month period, while being easy to place and retrieve. Another advance is that the present invention can be effective at a smaller volume within the stomach than existing intragastric members, such as balloons.

The scope of the present invention is defined in the appended claims.

In one arrangement, the obesity treatment apparatus comprises an intragastric member that is re-configurable from a first configuration to a second configuration, the first configuration being sufficiently small to permit introduction of said intragastric member into a gastric lumen of a mammal, the second configuration being sufficiently large to prevent said intragastric device from passing through the mammal's pylorus, wherein said intragastric member comprises a plurality of spaced apart openings. The apparatus further comprises an elongate member having a proximal end and a distal end wherein the elongate member is threaded through the openings of the intragastric member. The intragastric member is disposed between the proximal end and distal end of the elongate member along a plurality of ribs extending between the proximal end and distal end of the elongate member. A distal stopper, such as a pawl, is engaged to the distal end of the elongate member for securing the intragastric member along the elongate member and a proximal stopper, such as a second pawl, is engaged to the proximal end of the elongate member for locking the intragastric member along the elongate member. The apparatus also comprises a cinching member, such as a nylon thread or similar thread-like structure, having a proximal end and a distal end wherein the proximal end is engaged to a distal end of the proximal stopper and the distal end is engaged to the proximal end of the elongate member. The cinching member is for moving the proximal stopper along the elongate member towards the distal stopper.

The elongate member of the apparatus can include a connector engaged to the proximal end of the elongate member wherein the connector comprises a lumen configured to receive the cinching member as it passes from a lumen of the proximal stopper. A second distal stopper, such as a drag, is engaged to the distal end of the elongate member between the distal stopper and the plurality of ribs while a second proximal stopper, such as a second drag, is engaged along the cinching member.

In yet another arrangement, the obesity treatment apparatus comprises an intragastric member that is re-configurable from a first configuration to a second configuration, the first configuration being sufficiently small to permit introduction of said intragastric member into a gastric lumen of a mammal, the second configuration being sufficiently large to prevent said intragastric device from passing through the mammal's pylorus, wherein said intragastric member comprises a plurality of spaced apart openings. The apparatus further comprises an elongate member having a proximal end and a distal end wherein the intragastric member is threaded along a plurality of ribs extending between the proximal end and the distal end of the elongate member. A distal stopper, such as a button, is engaged to the distal end of the elongate member for securing the intragastric member along the elongate member. A proximal stopper, such as a second button, is engaged to the proximal end of the elongate member for locating the intragastric member along the elongate member. The apparatus also comprises a cinching member, such as a nylon thread or similar thread-like structure, having a proximal end and a distal end. The proximal end of the cinching member is engaged to the proximal stopper and extends through a lumen of the elongate member wherein the distal end of the cinching member is engaged to the distal stopper.

The elongate member further includes a locking member engaged along a distal end of the elongate member, wherein the locking member engages the distal stopper upon delivery of the intragastric member into the gastric lumen. The locking member comprises a lumen to receive the cinching member as it passes through the lumen of the elongate member. The proximal stopper also comprises a lumen to receive the cinching member as it passes through the lumen of the elongate member. Additionally, the apparatus includes at least one second distal stopper engaged with the distal end of the elongate member to secure the intragastric member upon delivery into the gastric lumen. When the intragastric member is secured, the distal stopper is engaged to the proximal stopper by pulling the cinching member proximally to remove the cinching member from the lumen of the elongate member. In addition, the proximal stopper is engaged to the plurality of ribs of the elongate member by pulling the cinching member proximally to remove the cinching member from the lumen of the elongate member.

In yet another arrangement, the obesity treatment apparatus comprises an intragastric member that is re-configurable from a first configuration to a second configuration, the first configuration being sufficiently small to permit introduction of said intragastric member into a gastric lumen of a mammal, the second configuration being sufficiently large to prevent said intragastric device from passing through the mammal's pylorus. The apparatus further comprises a cinching member, such as a nylon thread or similar thread-like structure, having a proximal end and a distal end wherein the intragastric member is threaded between the proximal end and the distal end of the cinching member. A distal stopper is engaged to the distal end of the cinching member for securing the intragastric member along the cinching member. A proximal stopper and a second proximal stopper are engaged to the proximal end of the cinching member for locking the intragastric member along the cinching member.

The distal stopper comprises a first lumen and a second lumen to receive the cinching member as it is passes from the proximal stopper, The proximal stopper also comprises a lumen for receiving the cinching member as it passes from the distal stopper. The second proximal stopper comprises a first lumen, a second lumen and a third lumen for receiving the cinching member as it passes from the distal stopper. When the intragastric member is secured, the proximal stopper is engaged to the second proximal stopper by pulling the cinching member proximally through the second lumen and third lumen of the second proximal topper. In this embodiment, the distal stopper and the proximal stopper can also include a pair of preformed expandable stoppers having an umbrella-like shape.

In another arrangement, the obesity treatment apparatus comprises an intragastric member that is re-configurable from a first configuration to a second configuration, the first configuration being sufficiently small to permit introduction of said intragastric member into a gastric lumen of a mammal, the second configuration being sufficiently large to prevent said intragastric device from passing through the mammal's pylorus. The apparatus further comprises a first cinching member having a proximal end and a distal end wherein the intragastric member is threaded between the proximal end and the distal end of the first cinching member. The apparatus also comprises a second proximal stopper, such as a bead, having a proximal end and a distal end, wherein the proximal end is engaged to a second cinching member and the distal end is engaged to the first cinching member. A distal stopper is engaged to the distal end of the first cinching member for securing the intragastric member along the first cinching member while a proximal stopper is engaged to the proximal end of the first cinching member for locating the intragastric member along the first cinching member. The first cinching member and second cinching member can comprise a nylon thread or similar thread-like structure.

The second proximal stopper further comprises a first lumen for receiving the first cinching member as it passes from the distal stopper and a second lumen for receiving the second cinching member as it passes from the proximal stopper. The distal stopper comprises a first lumen, a second lumen and a third lumen, wherein the first lumen receives the first cinching member as it passes through the second lumen and the third lumen to engage the second proximal stopper. The distal stopper also comprises a fourth lumen for securing a wire guide to the distal stopper to facilitate delivery into the gastric lumen. The proximal stopper comprises a first lumen and a second lumen for receiving the second cinching member as it passes from the second proximal stopper, wherein the first lumen comprises a first diameter and the second lumen comprises a second diameter. The first diameter is smaller than the second diameter for securing the bead member in the first lumen of the proximal stopper. When the intragastric member is secured, the second proximal stopper is engaged to the proximal stopper by pulling the second cinching member proximally through the first lumen of the proximal stopper. In addition, the first diameter allows the second proximal stopper to pass distally through the first lumen and prevents the second proximal stopper from passing proximally through the first lumen of the proximal stopper.

In yet another arrangement, the obesity treatment apparatus comprises an intragastric member that is re-configurable from a first configuration to a second configuration, the first configuration being sufficiently small to permit introduction of said intragastric member into a gastric lumen of a mammal, the second configuration being sufficiently large to prevent said intragastric device from passing through the mammal's pylorus. The apparatus further comprises an outer delivery tube having a main lumen, a proximal end, and a distal end, wherein the intragastric member is loaded between the proximal end and distal end of the outer delivery tube in the first configuration. An inner delivery tube is engaged with the main lumen of the outer delivery tube and at least one stopper is engaged with the inner delivery tube to secure the intragastric member upon delivery into the gastric lumen. The apparatus also includes an overtube comprising a proximal end, a distal end, and a lumen configured to receive the intragastric member in the first configuration for delivery into the gastric lumen wherein the intragastric member is expanded to the second configuration. The overtube comprises a groove extending about and around the surface of the overtube wherein the groove is in communication with a plurality of rollers to facilitate delivery of the intragastric member into the gastric lumen.

In yet another arrangement, the intragastric member can comprise a single strip of material having a series of apertures spaced along the length thereof, wherein the strip of material is bundled into a series of folds by passing a cinching member through the apertures and cinching the strip of material together. The intragastric member is inserted into the gastric lumen by passing the apertures of the strip of material over a wire guide, preferably in separate bundles, until the entire strip has been accumulated and bundled together inside the gastric lumen with a cinching member. The cinching member can be cut to allow the bundles to separate, thereby facilitating its removal by grasping and pulling one end of the strip.

In another arrangement, the obesity treatment apparatus comprises an intragastric member having a plurality of openings extending along the surface of the intragastric member to reduce the mass of the intragastric member. The intragastric member can also include a folded edge, wherein the folded edge engages a roller mechanism to facilitate the delivery of the intragastric member into the gastric lumen.

In yet another arrangement, the obesity treatment apparatus comprises a cutting member having a handle portion engaged to a proximal end of the cutting member and a wire position engaged to a distal end of the cutting member. The wire portion of the cutting member comprises a hook for cutting or removing a suture of an intragastric member. The cutting member is used to facilitate the removal of the intragastric member.

The intragastric member can also include one or more elements selected from the group consisting of plastic, nylon, polyesters, polyurethanes, polyethylenes, polyamides, silicone and biocompatible polymers to which food will generally not adhere. The intragastric member may also comprise a continuous strip of material that has been folded to form a plurality of loops, said plurality of loops being connected together to form a shape suggestive of a butterfly or bow-tie.

These and other advantages, as well as the invention itself, will become apparent in the details of construction and operation as more fully described below. Moreover, it should be appreciated that several aspects of the invention can be used with other types of intragastric devices or procedures used for the treatment of obesity.

### BRIEF DESCRIPTION OF SEVERAL VIEWS OF THE DRAWINGS

Several embodiments of the present invention will now be described by way of example with reference to the accompanying drawings, in which:

**FIG. 1** depicts a pictorial view of an intragastric member;

**FIG. 2** depicts a pictorial view of the embodiment of FIG. 1 with an exemplary delivery system;

**FIG. 3** depicts a sectional view of the delivery system of FIG. 2;

**FIGS. 4-5** depicts a pictorial view of a pair of intragastric members of the present invention prior to, and after being coupled together;

**FIGS. 6-7** depict detail views of different embodiments of indigestible members of intragastric members;

**FIG. 8** depicts a partially sectional side view of an expandable intragastric member;

**FIG. 9** depicts a pictorial view of an intragastric member of the present invention being delivered from an outer catheter;

**FIG. 10** depicts a pictorial view of an intragastric member of the present invention that includes a splittable outer sheath;

**FIG. 11** depicts a side view of an intragastric member encased in a dissolvable outer package;

**FIG. 12** depicts a pictorial view of an intragastric member being manipulated by an endoscopic device;

**FIG. 13** depicts a set of intragastric members bundled together by a coupling mechanism;

**FIG. 14** depicts a schematic cross-sectional view taken along line 1414 of FIG. 13;

**FIG. 15** depicts a pictorial view of another embodiment of an intragastric member;

**FIG. 16** depicts a pictorial view of the embodiment of FIG. 15 separated into separate bundles and ready for insertion into the gastric lumen;

**FIG. 17** depicts a portion of the strip material that is used to form the embodiment of FIG. 15;

**FIG. 18** depicts the insertion of the separate bundles of FIG. 16 being inserted into the gastric lumen;

**FIG. 19** depicts a pictorial view of yet another embodiment of an intragastric member;

**FIG. 20** depicts a pictorial view of the embodiment of FIG. 19 separated into separate bundles and ready for insertion into the gastric lumen;

**FIG. 21** depicts a portion of the strip material that is used to form the embodiment of FIG. 19;

**FIG. 22** depicts the insertion of the separate bundles of FIG. 20 being inserted into the gastric lumen;

**FIG. 23** depicts a pictorial view of yet another embodiment of an intragastric member;

**FIGS. 24-25** depict an alternative method of inserting an intragastric member of the present invention into the gastric lumen;

**FIG. 26** depicts a pictorial view of yet another embodiment of an intragastric member;

**FIG. 27** depicts a stopper and cinching member used for the insertion of bundles into the gastric lumen;

**FIG. 28-29** depicts an inner delivery tube and outer delivery tube used for the insertion of bundles into the gastric lumen;

**FIG. 30** depicts digestive-resistant material threaded onto an outer delivery tube for insertion into the gastric lumen;

**FIG. 31** depicts a delivery tip of the outer delivery tube of FIG. 30;

**FIG. 32-33** depicts the insertion of separate bundles of FIG. 30 into the gastric lumen;

**FIG. 34** depicts a portion of the strip material that is used to form the embodiment of FIG. 30;

**FIG. 35** depicts a partial, cross-sectional view of a flexible overtube according to an embodiment;

**FIG. 36** depicts a partial sectional view of a flexible overtube according to an embodiment;

**FIG. 37** depicts a perspective view of a flexible overtube according to an embodiment;

**FIG. 38** depicts a rear perspective view of the valve of FIG. 36;

**FIG. 39** depicts a front perspective view of the valve of FIG. 36;

**FIG. 40** depicts a front side view of the valve of FIG. 36;

**FIG. 41** depicts a sectional view taken along line C-C' of FIG. 40;

**FIG. 42** depicts a sectional, perspective view of the flexible overtube of FIG. 36 having a reinforcement member;

**FIG. 43** depicts a partial, cross-sectional view showing the flexible overtube of FIG. 35 positioned in the mouth and along the esophagus of a patient such that the overtube distal end is positioned in the gastric lumen of the stomach;

**FIGS. 44-45** depicts a pictorial view of yet another embodiment of an intragastric member;

**FIGS. 46-47** depicts a perspective view of an umbrella stopper of the intragastric member of FIG. 45;

**FIG. 48** depicts a perspective view of an overtube having a plurality of rollers along the lumen of the overtube;

**FIG. 49** depicts a sectional view of an overtube having a plurality of grooves along the lumen of the overtube;

**FIG. 50** depicts a partial sectional view of an overtube having a plurality of grooves along the lumen of the overtube;

**FIG. 51** depicts a pictorial view of an overtube having a plurality of rollers along the surface of the overtube for delivery of an intragastric member;

**FIG. 52** depicts a pictorial view of a distal tip of the overtube of FIG. 51;

**FIG. 53** depicts a pictorial view of another embodiment of a distal tip of the overtube of FIG. 51;

**FIG. 54** depicts a pictorial view of a chain and pulley gear mechanism utilized with the overtube of FIG. 51;

**FIG. 55** depicts a pictorial view of another embodiment of an overtube having a plurality of rollers along the surface of the overtube for delivery of an intragastric member;

**FIG. 56** depicts a pictorial view of an intragastric member wherein the intragastric member comprises a folded edge;

**FIG. 57** depicts a pictorial view of the intragastric member of FIG.56 being delivered with a roller mechanism;

**FIG. 58** depicts a pictorial view of a cutting member;

**FIG. 59** depicts a side view of the cutting member of FIG. 58;

**FIG. 60** depicts a sectional view of the cutting member of FIG. 58 extended from a sheath;

**FIG. 61** depicts a sectional view of the cutting member of FIG. 60 retracted into the sheath;

**FIG. 62** depicts a pictorial view of an intragastric member wherein the intragastric member comprises a plurality of openings;

**FIG. 63** depicts a pictorial view of an embodiment of an elongate member comprising a cinching member;

**FIG. 64** depicts a pictorial view of the elongate member of FIG. 63 threaded with an intragastric member;

**FIG. 65** depicts a pictorial view of the elongate member of FIG. 64 secured to the intragastric member upon delivery into the gastric lumen;

**FIG. 66** depicts a pictorial view of another embodiment of an elongate member comprising a cinching member;

**FIG. 67** depicts a pictorial view of the elongate member of FIG. 66 threaded with an intragastric member;

**FIG. 68** depicts a pictorial view of the elongate member of FIG. 67 secured to the intragastric member upon delivery into the gastric lumen;

**FIG. 69** depicts a pictorial view of another embodiment of a cinching member;

**FIG. 70** depicts a pictorial view of the cinching member of FIG. 69 secured to an intragastric member;

**FIG. 71** depicts a pictorial view of another embodiment of a first cinching member and a second cinching member comprising and an intragastric member threaded to a delivery device during delivery into the gastric lumen;

**FIG. 72** depicts a pictorial view of the first cinching member and the second cinching member of FIG. 71 attached to the intragastric member during delivery into the gastric lumen;

**FIG. 73** depicts a pictorial view of the first cinching member and the second cinching member of FIG. 71 attached to a second locking member during delivery into the gastric lumen;

**FIG. 74** depicts a pictorial view of the first cinching member of FIG. 71 secured to the intragastric member upon delivery into the gastric lumen;

**FIG. 75** depicts a pictorial view of a second proximal stopper engaged to the first cinching member and the second cinching member of FIG.

**FIG. 76** depicts a distal view of a distal stopper of FIG. 73

**FIG. 77** depicts a proximal view of the distal stopper of FIG. 73;

**FIG. 78** depicts a distal view of a proximal stopper of FIG. 73;

**FIG. 79** depicts a distal view of the proximal stopper of FIG. 73 wherein the proximal stopper is engaged to the second proximal stopper; and

**FIG. 80** depicts a proximal view of the proximal stopper of FIG. 74 wherein the second proximal stopper is secured to the proximal stopper.

### DETAILED DESCRIPTION OF THE INVENTION

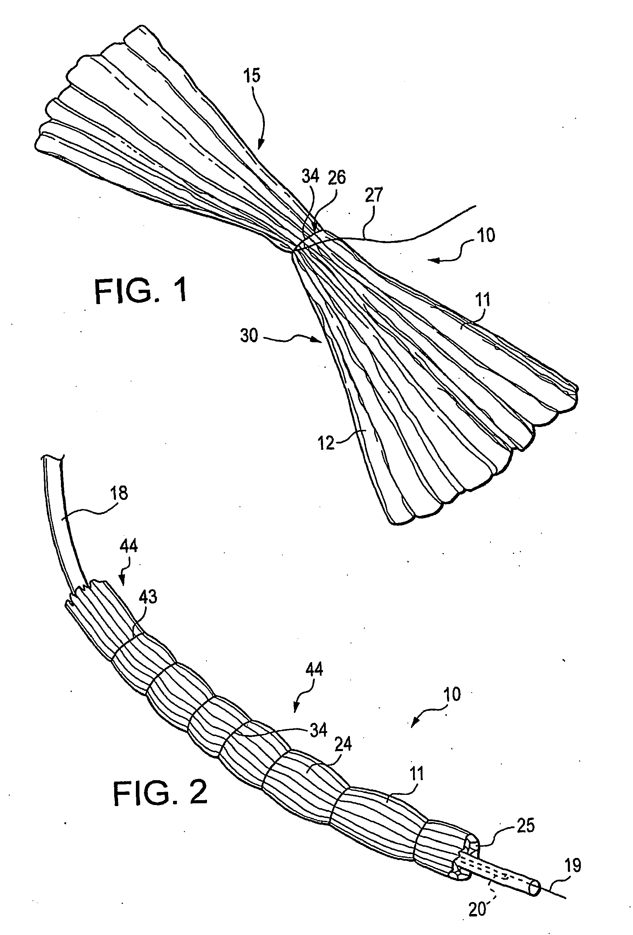

The obesity treatment apparatus 10 depicted in **FIGS.** 1-25 comprises one or more intragastric members **11,** each comprising one or more digestive-resistant or indigestible member **12** sized and configured such that the intragastric member **11** can be placed into the stomach of a mammalian patient and reside therein, and being generally unable to pass through the pylorus. As used herein, the terms digestive-resistant and indigestible are intended to mean that the material used is not subject to the degradative effects of stomach acid and enzymes, or the general environment found within the gastric system over an extended period of time, therefore allowing the device to remain intact for the intended life of the device. This does not necessarily mean that the material cannot be degraded over time; however, one skilled in medical arts and gastrological devices would readily appreciate the range of material that would be suitable for use as a long-term intragastric member.

Many well-known plastics have suitable properties, including selected polyesters, polyurethanes, polyethylenes, polyamides, silicone, or other possible materials. Mammalian hair has been found to form natural bezoars, and thus, is also a possible material. However, some materials, such as certain polyamides, have been found to expand over time, which can be an undesirable property. Most other natural materials are generally much less resistant to acids and enzymes, and would therefore typically require treatment or combination with resistant materials to function long term, unless a shorter-term placement is intended or desired.

In the preferred embodiments, the digestive-resistant or indigestible member 12 comprises a low density polyethylene having a thickness of about 40-50 microns. Fluorinated ethylene propylene, ethylene vinyl acetate copolymer, nylon, or types of polymers that are biocompatible and to which food will generally not adhere may also be utilized.

**FIG. 1** depicts a single intragastric member **11** in which the digestive-resistant members **12** include a plurality of elongate plastic strips **30** that are secured together in the middle by a retaining element **34,** such as a nylon thread. The thread can be elongated to serve as a coupling mechanism **26,** such as a tether **27.** The number of digestive-resistant members **12** or strips **30** used to form the intragastric member **11** depends on the material used, their length and width, and how many intragastric members **11** comprise a set or grouping. The optimal length of the intragastric member **11** is determined by considering these same factors, as well by what is determined through experimentation to work best.

Feasibility studies have been primarily limited to placement in pigs with both 8 cm and 16 cm intragastric members being used, both having a total volume of about 40 ml when placed in the stomach of the animal. Although the experiments were designed to establish the safety of the device, significant weight loss was nevertheless observed in the test animals. Although no gastric ulcers were found in animals with polyester intragastric members, there was a 20% incidence of gastric ulcers in animals having polyamide devices.

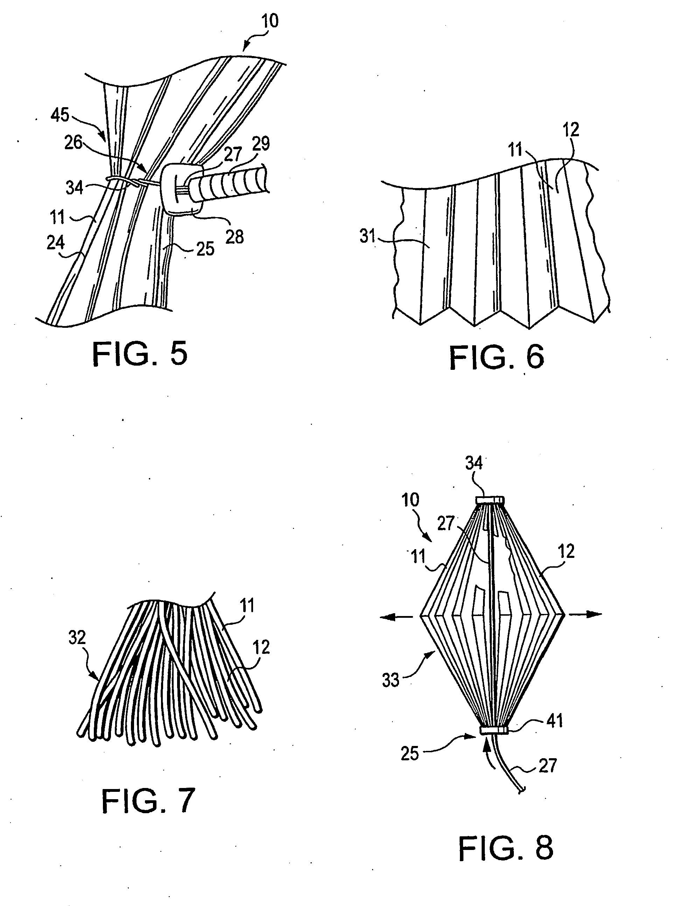

Results from human trials may lead to modifications in the configuration being depicted in the figures of this application. Nevertheless, it is already understood that the dimensions shape, and construction of the intragastric member can be quite variable and still produce the desired results. For example, **FIGS. 6-7** depict an alternative digestive-resistant member 12. In the embodiment shown in **FIG. 6****,** the strips **30** of **FIG.** 1 are replaced by digestive-resistant member **12** comprising a folded or pleated sheet **31** of plastic or other material. Either a single sheet **31** or multiple sheets can be used to form the intragastric member **11** of this embodiment. The embodiment shown in **FIG. 7** depicts an intragastric member **11** in which the digestive-resistant members **12** comprise a plurality of elongated fibers or hairs **32,** typically made of polymer or other synthetic material.

In the illustrative embodiments, the retaining element **34** (see **FIG. 1****)** is located about the center of the device to hold the digestive-resistant members **12** together. However, a skilled artisan would appreciate that other designs utilizing differently placed retaining elements **34,** or eliminating them entirely, could also be utilized. For example, **FIG. 8** depicts an expandable device **33** that comprises a retaining element **34** at one end to secure the digestive-resistant members **12,** which in this embodiment are typically made of a material having a certain degree of stiffness. The other end is secured by a second, slidable retaining element **41** that is disposed over a tether **27** attached to the first retaining member **34.** The intragastric member **11** is deployed in an elongated configuration with the retaining elements **34, 41** located near their maximum possible difference apart. After the device is placed in the gastric lumen, the slidable retaining element **41** is urged along the tether **27** and toward the first retaining element **34** by using a tube, probe, or other device, until the digestive-resistant members **12** have bowed outward, thus increasing the overall dimensions and volume of the device. The slidable retaining element **41** continues to grip the tether **27** after the urging mechanism is removed, retaining the increased dimensions of the intragastric member **11** until further manipulation is needed to reduce its diameter for removal from the patient.

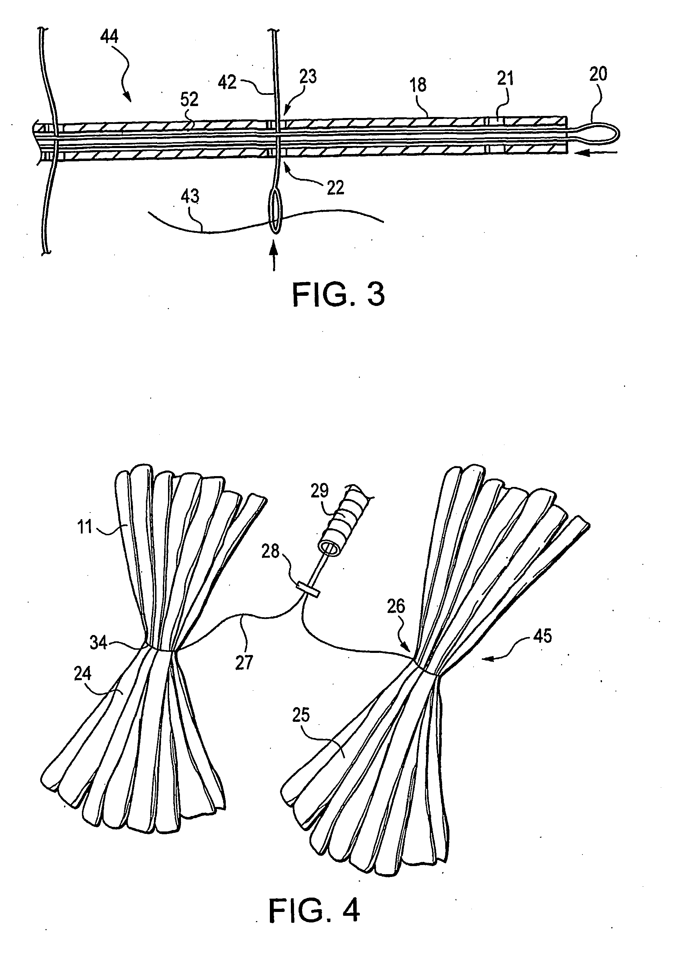

Deployment of intragastric member **11** can be accomplished in a number of ways, depending on the size, number, and configuration of the devices, or according to physician or patient preference. **FIGS. 2-4** depict one such delivery system **44** in which first and second intragastric members **24, 25** are mounted over a plastic overtube **18** and secured by a series of suture ties **43,** such as cotton thread. A wire guide **19** is typically used in the procedure, and is placed through the passageway **52** of the overtube **18.** As shown in **FIG. 3****,** the overtube **18** includes a plurality of apertures **21,** a pair of which (e.g., apertures **22** and **23)** are distributed approximately every 2 cm along the distal portion of the overtube **18.** To secure the intragastric members **24, 25,** the suture tie is pulled through the first aperture **22** using a device **42** such as a loop, hook, snare, etc. It is fed through a releasing mechanism **20,** such as the illustrative wire loop, and then pulled through the opposite aperture **23.** The intragastric members **24, 25** are then placed on the overtube **18,** and the suture ties **43** are secured, thereby constraining the intragastric members into a first configuration **14** for delivery. Once the delivery system **44** has been introduced into the gastric lumen, the releasing mechanism **20** is pulled back through the overtube **18,** thereby severing the suture ties **43** one by one and releasing the intragastric members **11** into the gastric lumen where they can assume a second configuration **10** (see **FIG. 1****)** that is sufficiently voluminous such that they cannot pass from the stomach.

In order to create an obesity treatment apparatus **10** that will be retained in the stomach, it may be necessary that the intragastric members **11** be coupled together to form a grouping or set **45** of intragastric members. **FIG. 4** shows the two deployed intragastric members **24, 25** that each have a coupling mechanism **26** (tether **27)** attached about them such that they can be drawn together as depicted in **FIG. 5****.** A push member **29,** such as a corrugated metal tube, is placed into gastric lumen by using an endoscope, and is guided over the tethers **27** to urge a securing element **28,** such as a rubber patch, tightly against the two intragastric members **24, 25.** The tethers **27** can then be cut, allowing the grouping **45** to float free within the stomach. This method can also be used to join additional intragastric members **11** to form a larger grouping **45.** Likewise, the illustrative delivery system **44** of **FIG. 2** can be used to deliver any practical number of intragastric members **11,** which can then be joined in the manner described above, or they can be delivered singly or in pairs, and then grouped together after all of the intragastric members **11** have been placed.

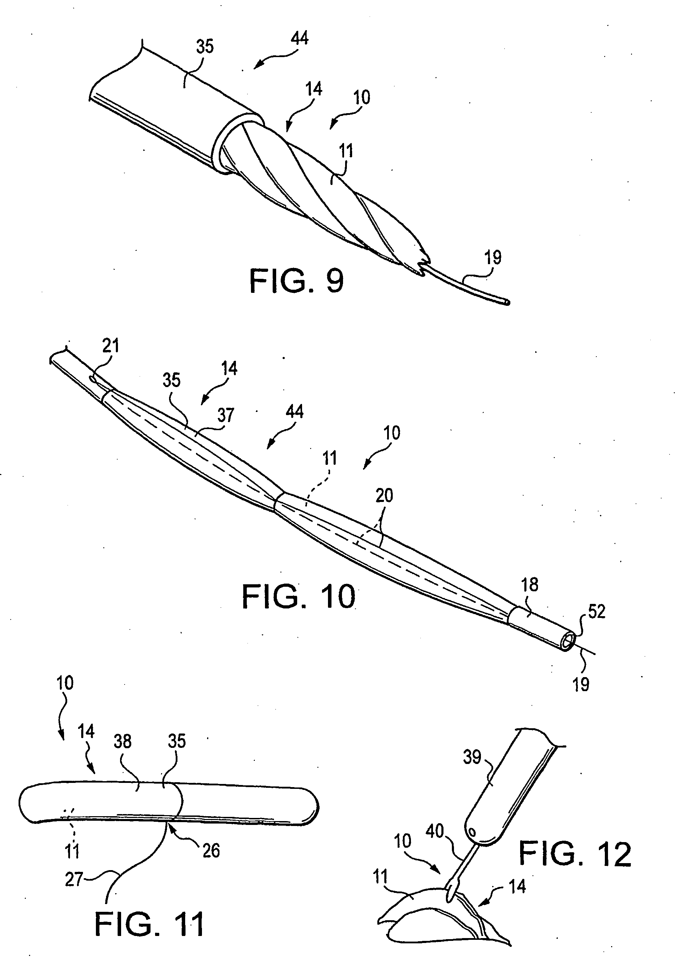

**FIGS. 9-11** depict intragastric members **11** that are delivered into the gastric lumen within an outer member **35,** such as a sheath, tube, package, wrapping, etc., and subsequently released. For example, **FIG. 9** depicts a delivery system **44** in which the intragastric member **11** (or multiple devices) is preloaded into an outer tube or introducer, then deployed therefrom by being pushed out by using a pusher member (not shown). The intragastric member **11** is shown twisted to aid in loading and deployment.

**FIG. 10** depicts a delivery system 44 in which the intragastric member is loaded over a tube **18** (as in **FIG. 2****),** but is secured by an outer member **35** comprising a splittable sheath **37** or sleeve made of a thin plastic material. In the illustrative embodiment, the releasing mechanism **20** comprises a nylon thread or wire that is looped under and over the sheath **37,** such that it can be withdrawn to tear through the thin material of the sheath **37** to release the intragastric member(s) **11** mounted on the tube **18.** The releasing mechanism of **FIG. 10** feeds into an aperture **21** and passageway **52** of the tube **18,** where it extends to the proximal end of the apparatus **10.** Other types of splittable sheaths **37** can also be used, such as the COOK® PEEL-AWAY Introducer Sheath.

**FIG. 11** depicts an intragastric member **11** that includes an outer member **35** comprising a dissolvable enclosure **38**. The material, such as cellulose, gelatin, or some other dissolvable or rapidly degrading synthetic or biomaterial material, allows the intragastric member **11** to be deployed in the first configuration **14** into the stomach, where it expands into the second configuration **15** (see, e.g., **FIG.** 1) once the outer enclosure **38** has dissolved or degraded away. The embodiment of **FIG. 11** can be delivered with or without a catheter-based delivery system **44,** or swallowed by the patient, depending on the outer dimensions of the apparatus **10.**

**PIG. 12** also depicts a method of delivering the apparatus **10** without a catheter or tube **18.** It has been found that the intragastric members **11** can be pulled into the gastric lumen using an endoscope **39** and endoscopic instrument **40,** such as a forceps, basket, snare, etc. This technique can be employed to pull groupings **45** (see, e.g., **FIG. 4****)** of intragastric members **11** into the gastric lumen, as long as the alimentary tract is sufficiently wide to accommodate the grouping **45.**

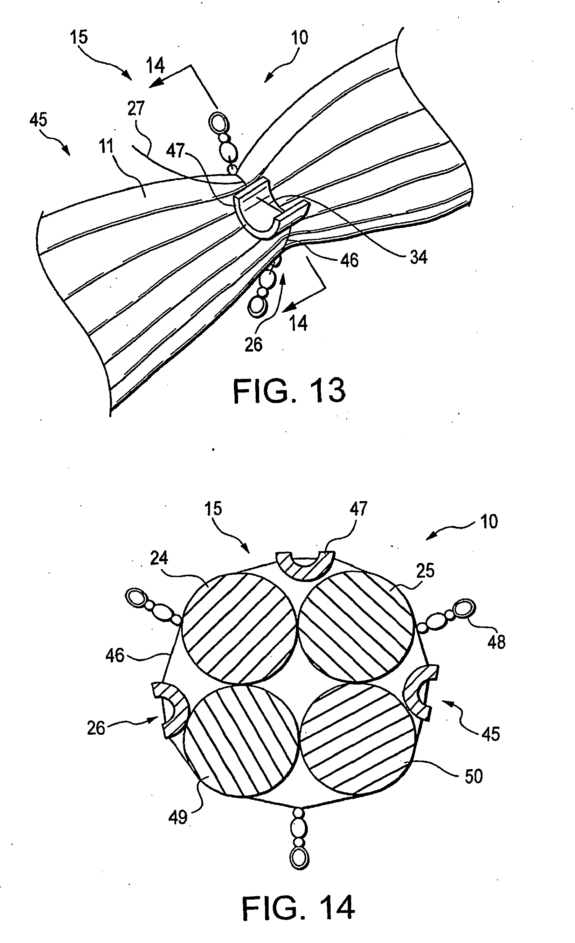

**FIGS. 13-14** depict a grouping **45** of four intragastric members **24,25,49,50** that are pre-coupled to one another by a coupling mechanism **26** prior to introduction into the gastric lumen. Although such an arrangement or grouping **45** is sufficiently small such that it can be introduced into the gastric lumen as a set, the adherence of mucous and other changes that occur within the stomach environment can, over time, significantly increase the volume of the apparatus **10** from, for example, an original size of about 60 ml up to a possible size of about 150 ml. The increased size can make it very difficult to remove the grouping **45** from the stomach. To address this problem, multiple intragastric members **45** are grouped together for introduction, and then cut apart when it is time to remove them from the patient. The coupling mechanism **26** comprises a grouping mechanism **46,** such as a nylon thread (e.g., standard nylon fishing line), that is wrapped around the grouping **45** to pull them into close contact with one another. The grouping is released by severing the line comprising the grouping mechanism **46** and the intragastric members **24,25,49, 50** are removed one at time using a retrieval device such as that shown in **FIG. 12****.**

To assist the operator in cutting the line **46** to release the grouping **45,** two different coupling components **47, 48** are included in the illustrative embodiment. The first coupling component **47** comprises a curved polymer piece which is traversed by the line **46** in such a manner that the line **46** can be readily visualized under the scope, thereby providing a place to grasp and/or cut the line with an instrument extending from the endoscope. The second coupling component **48** comprises a fishing line swivel, which being metal, can be readily visualized, as well as providing a hard surface against which a cutting device can be applied to sever the line **46,** especially if the line has proved difficult to cut using other methods. It also provides an easily accessible point on the apparatus **10** which can be grabbed with a forceps or other device.

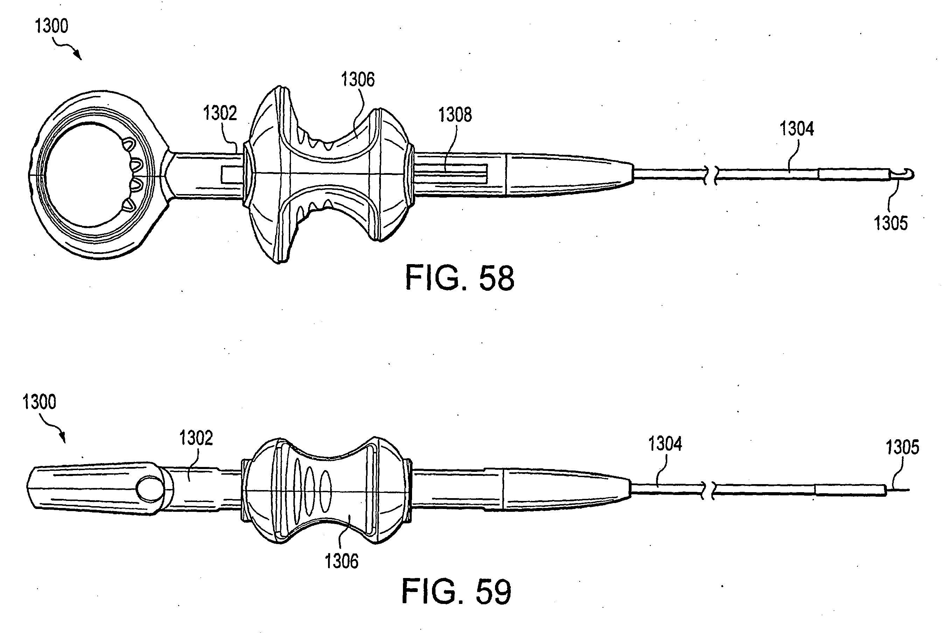

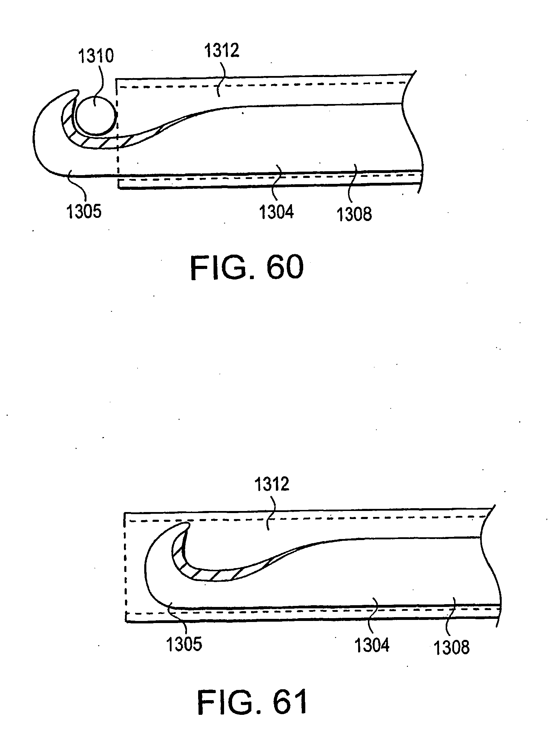

For example, **FIGS. 58-61** depict a cutting device **1300.** The cutting device 1300 is utilized for cutting and removing the intragastric member after insertion into the gastric lumen of the patient. The cutting device **1300** comprises a proximal end **1302** and a distal end **1304,** wherein a handle member **1306** extends along the proximal end **1302** and a wire portion **1308** extends along the distal end **1304** of the cutting device **1300.** The wire portion **1308** comprises a hook **1305** engaged to the distal end of the wire portion **1308.** The hook **1305** is utilized to remove a
suture **1310** or tubing of the intragastric member. The cutting device **1300** can be inserted into a sheath **1312** and delivered through an endoscope into the gastric lumen. During use, the hook **1305** of the cutting device **1300** is extended from the sheath **1312** positioned about the suture **1310,** and then is retracted, which results in the cutting of the suture **1310 (****FIG. 60-61****).** The sheath **1312** provides a protective layer to secure the hook **1305** from damaging the lumen of the endoscope or other delivery device.

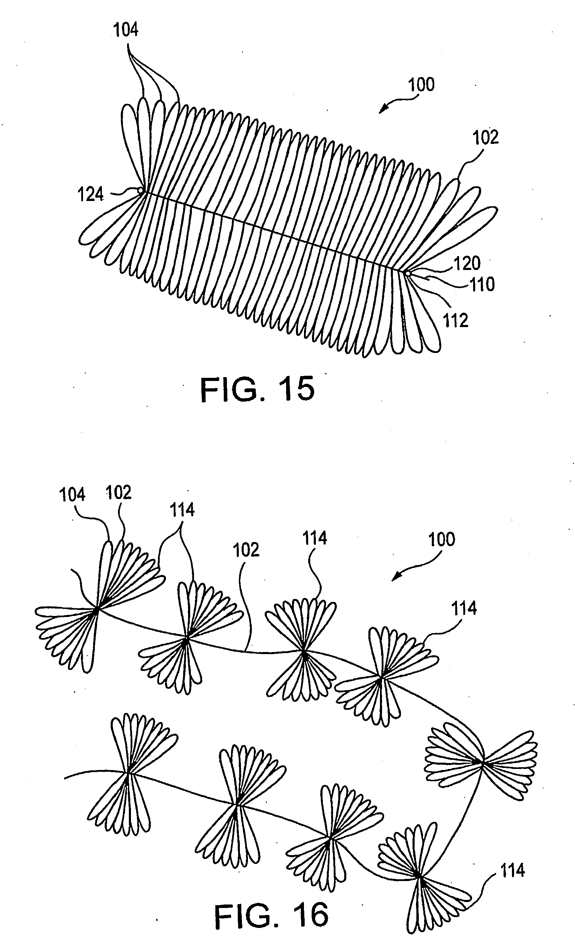

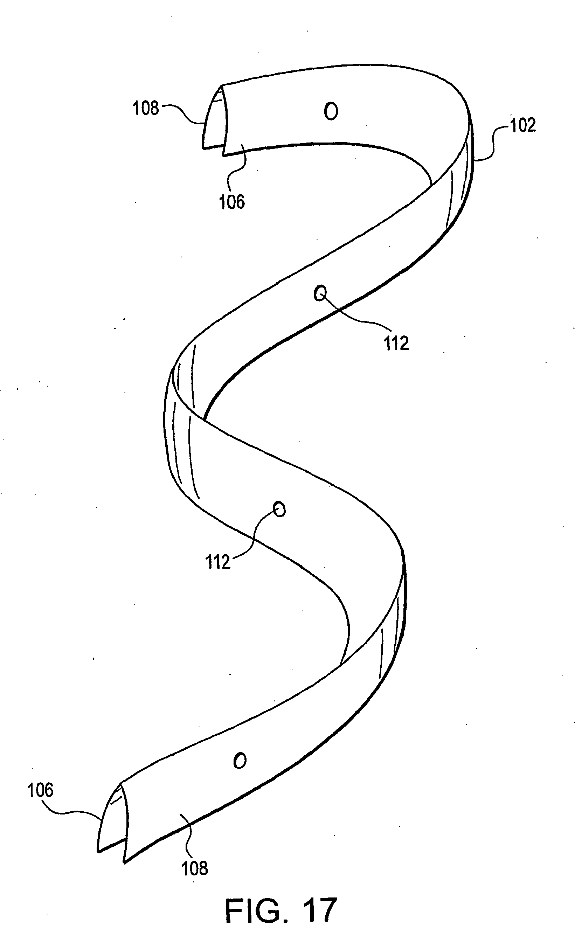

**FIG. 15** depicts another embodiment of an intragastric member **100.** In this embodiment, the intragastric member **100** comprises a single strip of high-density polyethylene **102** that has been folded and bundled to form eighty-nine (89) loops **104** in the general shape of a butterfly or bow-tie. As best seen in **FIG. 17****,** the single strip of high-density polyethylene **102** of the embodiment is formed from a tube of material having a wall thickness of 40-50 microns and a perimeter of 3 cm that has been sliced in half. Each half of the material is then folded to form a strip **102** having two walls **106,108,** wherein each wall **106,108** has a width of 1.5 cm. Of course, the strip **102** could comprise a different number of walls **106, 108,** have a different width and thickness, or be formed from a tube of material.

In the embodiment of the intragastric member **100** shown in **FIG. 15****,** each loop **104** is 20 cm in length. Accordingly, the intragastric member **100** is formed from single strip **102** having a total length of approximately 35.6 m.

The intragastric member **100** is bundled by passing a cinching member **110** through an aperture **112** in the strip **102** at the center of the each loop **104.** In this embodiment, the cinching member **110** comprises a nylon thread or similar thread-like structure. As best seen in **FIG. 17****,** the apertures **112** are formed in each wall **106,108** of the strip **102,** and are spaced so that loops **104** are formed 20 cm in length when adjacent apertures **112** are pulled together to form the intragastric member **100** shown in **FIG. 15****.** In other words, the apertures **112** are located every 20 cm along the length of the strip **102.**

The embodiment of the intragastric member **100** shown in **FIG. 15** may be too large for delivery or insertion into the gastric lumen while in its bundled, final configuration. Accordingly, the intragastric member **100** is preferably inserted into the gastric lumen in stages. For example, and as shown in **FIG. 16****,** the intragastric member **100** is separated into nine (9) separate bundles **114,** each of which comprise approximately ten (10) loops **104** of the strip **102.** The loops **104** of each separate bundle **114** are temporarily grouped or held together by a twist tie **116** or similar device. Grouping the separate bundles **114** in this manner improves the handling of the material and prevents the strip **102** from becoming tangled or contaminated.

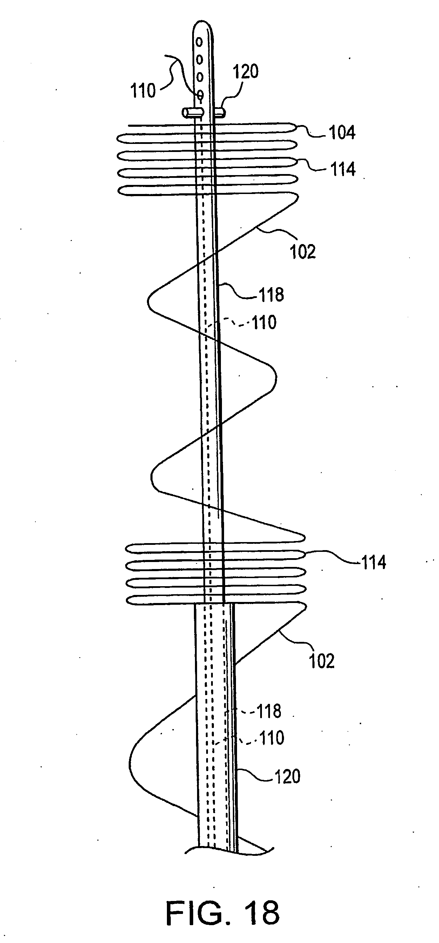

As shown in **FIG. 18****,** the separate bundles **114** of the intragastric member **100** are inserted into the gastric lumen one at a time by using a wire guide **118** such as a Savary-Gilliard™ wire guide, manufactured by Cook Endoscopy, Winston-Salem, N.C. The wire guide **118** comprises a central opening through which the cinching member **110** passes. The end of the cinching member **110** is connected to or tied around a small piece of nylon tubing **120** that is sized so as to not pass through the apertures **112** in the strip **102.** Prior to the insertion procedure, the nylon tubing **120** is placed near the distal (forward or insertion) end of the wire guide **118** so as to prevent the strip **102** of the first bundle **114** from sliding off the end of the wire guide **118.**

Once the distal end of the wire guide **118** is positioned in the gastric lumen, the first bundle **114** is threaded over the proximal (rearward) end by passing the apertures **112** over the wire guide **118.** A plastic tube **122** is then positioned over the proximal end of the wire guide **118,** and slid towards the distal end of the wire guide **118** so as to push the folds **104** of the first bundle against the nylon tubing **120.** This procedure is then repeated by threading subsequent bundles **114** over the wire guide **118** and pushing them against the previously inserted bundles **114** until all of the bundles **114** have been inserted into the gastric lumen. The bundles **114** are then secured together by pushing a small rubber stopper or similar device **124** (see **FIG. 15****)** along the wire guide **118** so as to press against the last bundle **114** to be inserted. The wire guide **118** is then withdrawn so as to leave the cinching member **110** extending through the apertures **112** of all of the bundles **114.** The cinching member **110** is then tied or otherwise secured to the stopper **124** so as to form a complete intragastric member **100** as shown in FIG. 15.

To remove the intragastric member **100** from the gastric lumen, the cinching member **110** is typically cut so as to release the folds **104.** One end of the strip **102** is then grasped by an endoscopic or similar device and pulled out of the patient.

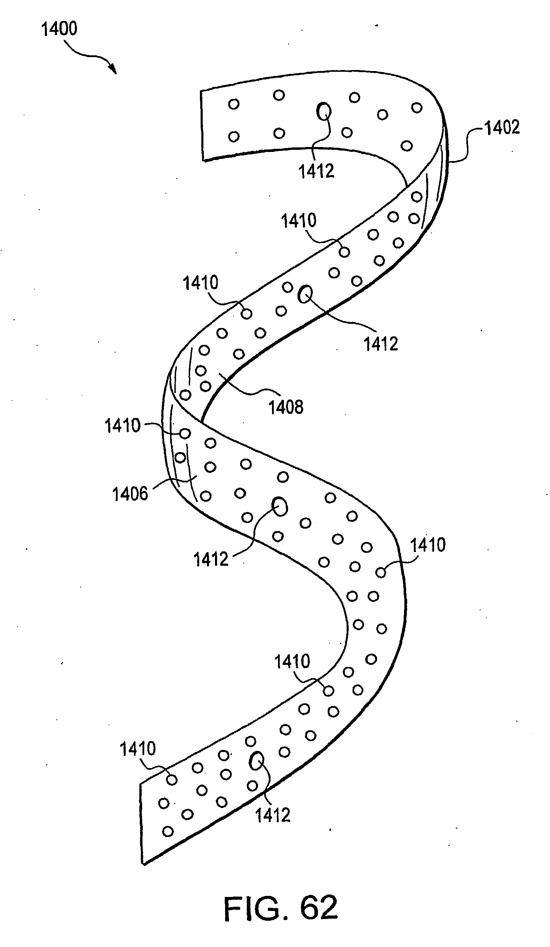

**FIG. 62** depicts another embodiment of an intragastric member **1400.** In this embodiment, the intragastric member **1400** comprises a single strip of high-density polyethylene **1402** having a plurality of openings **1410** positioned along the length of the strip **1402.** The plurality of openings **1410** reduce the overall mass of the intragastric member **1400** and also decreases the total thickness of the intragastric member **1400** after the strip **1402** is bundled in the gastric lumen. As best seen in **FIG. 62****,** the single strip of high-density polyethylene **1402** of the embodiment is formed from a sheet of material having a wall thickness of 7.5 microns and a perimeter of 6 cm. The single strip **1402** comprises a first side **1406** and an opposite side **1408** and is formed from a sheet of material. The intragastric member **1400** further comprises a plurality of apertures **1412** positioned along the center of the strip **1402.** The length of the intragastric member **1400** may vary depending on the particular shape and design.

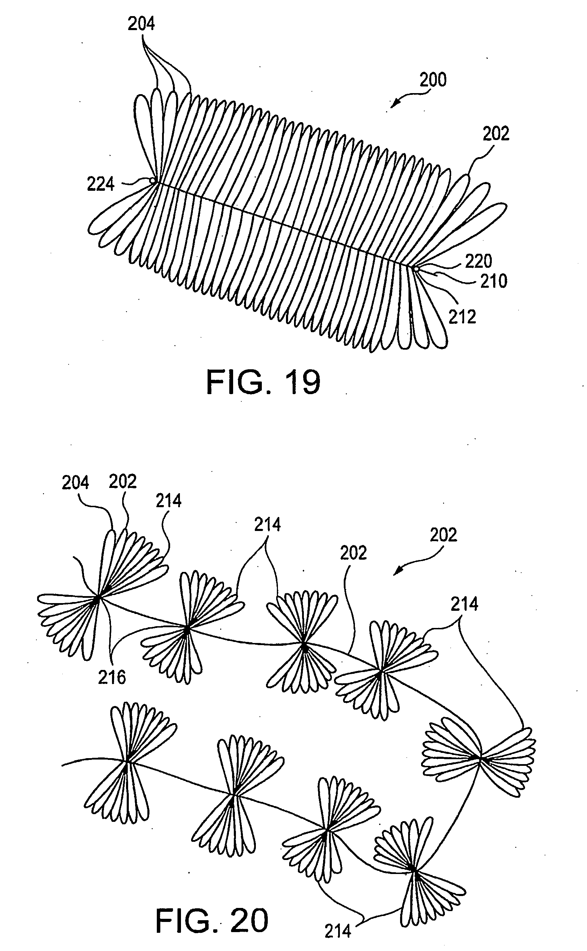

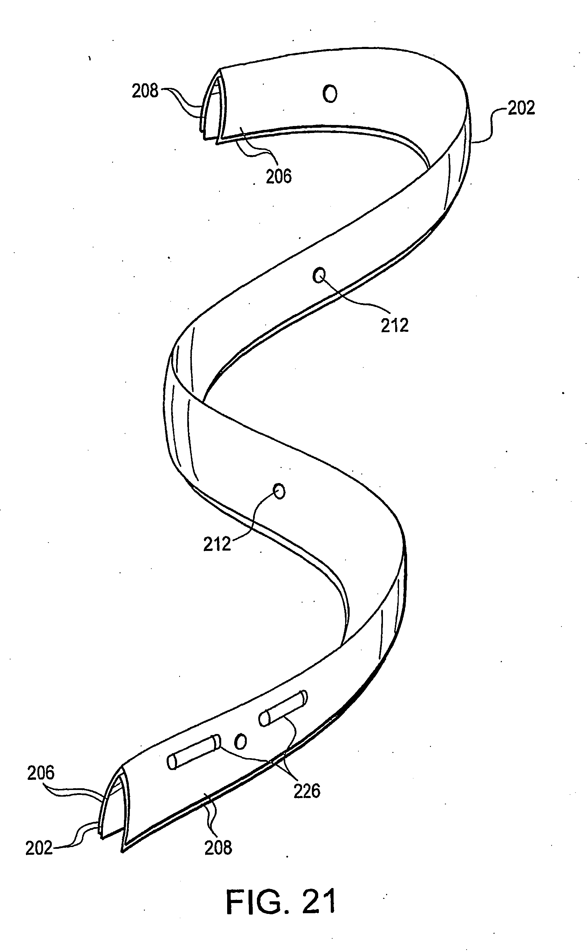

**FIG. 19** depicts yet another embodiment of an intragastric member **200.** In this embodiment, the intragastric member **200** comprises a double strip of low-density polyethylene **202** that has been folded and bundled to form approximately forty-five (45) loops **204** in the general shape of a butterfly or bow-tie. A double strip of low-density polyethylene **202** is defined as a first strip of low-density polyethylene bundled with a second strip of low-density polyethylene (see FIG. **21****).** The double strip of low-density polyethylene **202** of this embodiment comprises a pair of strips **202** each having two walls **206,208,** wherein each wall **206,205** has a width of 15mm and thickness in the range of 40-50 microns.

In the embodiment of the intragastric member **200** shown in **FIG. 19****,** each loop **204** is 20 cm in length. Accordingly, the intragastric member **200** is formed from a double strip **202** of material having a total length of approximately 18 m (i.e., each strip **202** has a total length of approximately 18 m). A double strip **202** having longer or shorter lengths may also be used depending on the desired size and mass of the intragastric member **200.**

The intragastric member **200** is bundled by passing a cinching member **210,** such as a nylon thread or similar thread-like structure, through an aperture **212** in each strip **202** at the center of the each loop **204.** As best seen in **FIG. 21****,** the apertures **212** are formed in each wall **206,208** of each strip **202,** and are spaced so that loops **204** are formed 20 cm in length when adjacent apertures **212** are pulled together to form the intragastric member **200** shown in **FIG. 19****.** In other words, the apertures **212** are located every 20 cm along the length of the strip **202,** In the preferred embodiment shown, apertures **212** have a diameter of approximately 3.5 mm.

The embodiment of the intragastric member **200** shown in **FIG. 19** may be too large for delivery or insertion into the gastric lumen while in its bundled, final configuration. Accordingly, the intragastric member 200 is preferably inserted into the gastric lumen is stages. For example, and as shown in **FIG. 20****,** the intragastric member **200** is separated into nine (9) separate bundles **214,** each of which comprise approximately five (5) loops **204** of the strip **202.** The loops **204** of each separate bundle **214** are grouped or held together by a breakable tie **216,** made of cotton thread, or similar device. As will be explained below, grouping the separate bundles **214** in this manner improves the handling of the material and prevents the strips **202** from becoming tangled or contaminated during the insertion thereof.

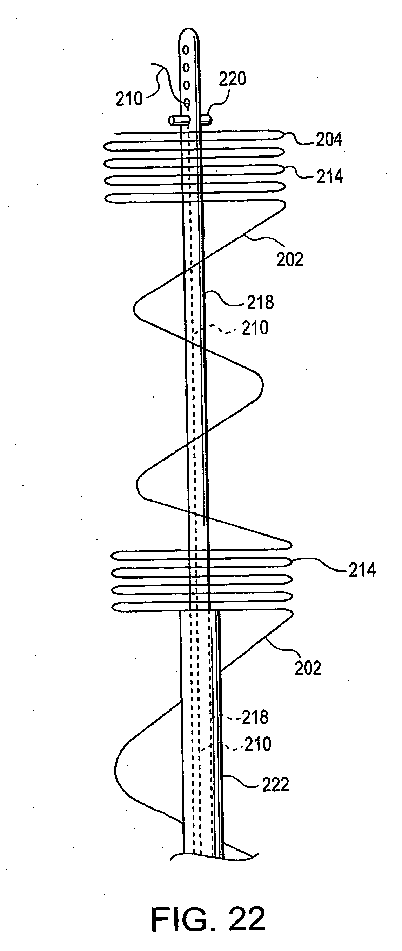

As shown in **FIG. 22****,** the separate bundles **214** of the intragastric member **200** are inserted into the gastric lumen one at a time by using a wire guide 218 such as a Savary-Gilliard™ wire guide, manufactured by Cook Endoscopy, Winston-Salem, N.C. The wire guide **218** comprises a central opening through which the cinching member **210** passes. The end of the cinching member **210** is connected to or tied around a small nylon disc **220** that is sized so as to not pass through the apertures **212** in the strips **202.** Prior to the insertion procedure, the nylon disc **220** is placed near the distal (forward or insertion) end of the wire guide **218** so as to prevent the strips **202** of the first bundle **214** from sliding off the end of the wire guide **218.**

Once the distal end of the wire guide **218** is positioned in the gastric lumen, the first bundle **214** is threaded over the proximal (rearward) end by passing the apertures **212** over the wire guide **218.** A pusher tube **222,** which may be plastic, metal or some other suitable material, is then positioned over the proximal end of the wire guide **218,** and slid towards the distal end of the wire guide **218** so as to push the folds **204** of the first bundle **214,** which remain bundled by tie **216,** against the nylon disc **220.**

In the preferred embodiment shown, one or more of the apertures **212** in each bundle **214** have an increased diameter that is sufficient to allow one more folds **204** to slide over the outside of the pusher tube **222.** This permits the portion of the strips **202** connected between adjacent bundles **214** to be guided (extended) along the wire guide **218** without interfering with the deployment of each bundle **214.** In the preferred embodiment shown, those apertures **212** having an increased diameter are approximately 9-10 mm in diameter.

This procedure is then repeated by threading subsequent bundles **214** over the wire guide **218** and pushing them against the previously inserted bundles **214** until all of the bundles **214** have been inserted into the gastric lumen. The bundles **214** are then secured together by pushing a small rubber stopper or similar device **224** (see **FIG. 19****)** along the wire guide **218** so as to press against the last bundle **214** to be inserted. The wire guide **218** is then withdrawn so as to leave the cinching member **210** extending through the apertures **212** of all of the bundles **214.** The cinching member **210** is then tied or otherwise secured to the stopper **224** so as to form a complete intragastric member **200** as shown in **FIG. 19****.**

To remove the intragastric member **200** from the gastric lumen, the cinching member **210** is typically cut so as to allow the intragastric member **200** to separate in separate bundles (see **FIG. 20****).** The separate bundles **214,** which remain connected to each other by strips **202,** can then be removed one at a time. In the event that the removal of the intragastric member **200** in separate bundles **214** becomes difficult or problematic, then breakable ties **216** may be severed to release the folds **204** of one or more of the bundles **216.**

As best seen in **FIG. 21****,** visual markers **226,** such as colored tubing, are sutured to the side of the strips **202** of the first or last fold **204** on either side of the aperture **212.** These markers **226** assist the physician in locating the cinching member **210,** which may be difficult to identify after the device has resided within the gastric lumen for an extended period of time. Once the cinching member **210** is cut, one end of the pair of strips 202, or one of the bundles **216,** is then grasped by an endoscopic or similar device and pulled out of the patient.

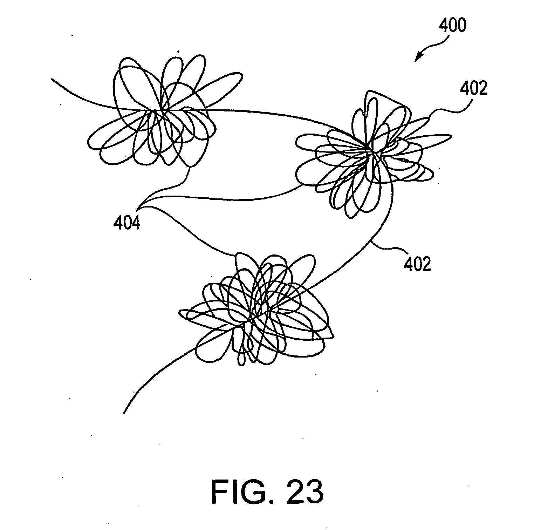

**FIG.** 23 depicts yet another embodiment of an intragastric member **400.** In this embodiment, the intragastric member **400** comprises nylon thread **402** or similar thread-like structure that has been tied into a series of nylon balls **404.** The nylon balls **404** are inserted into the gastric lumen separately and then connected together to form a single, larger mass of nylon thread (not shown).

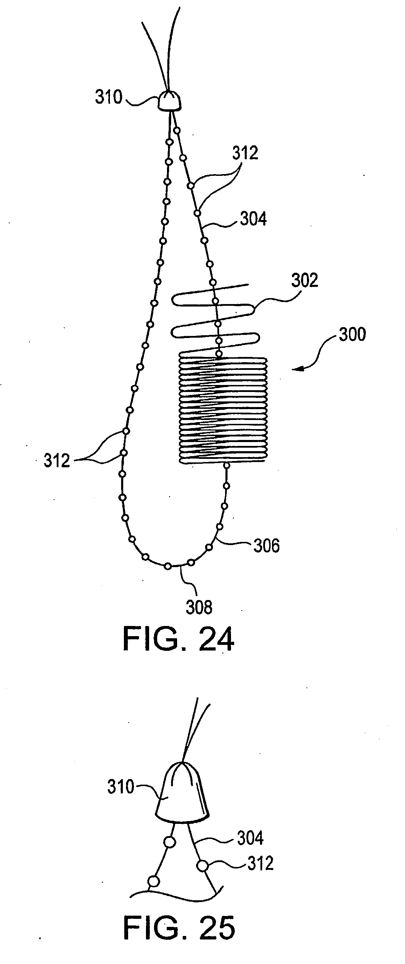

The above-described embodiments, particularly the embodiments of **FIGS. 15** and **19****,** can be deployed using alternative procedures. For example, and as shown in **FIGS. 24** and **25****,** the intragastric member **300** could be deployed by extending the strip 302 along a cinching member **304,** such as nylon thread or similar thread-like structure, that has been formed into a loop **306.** Once the end **308** of the loop **306** has been inserted into the gastric lumen, then a locating device 310, such as plastic cone (shown in detail in **FIG. 25****),** is pushed over both strands of the cinching member **304** so as to close the loop **306.** As the loop **306** is closed, the strip **302** is compressed so as to form an intragastric member **300** having a configuration similar to that shown in **FIGS. 15** and **19****.** Knots **312** are included along the cinching member **304** to provide a ratcheting action with the locking device **310.** After the intragastric member **300** has been deployed inside the gastric lumen, then the portion of the cinching member **304** extending beyond the locating device **310** can be severed with an endoscopic scissors and removed.

Alternatively, the strip **302** can be compressed by sliding a tube (not shown) along one or both halves of the loop **306.** In addition, the intragastric member **300** can be inserted in bundles (see **FIGS. 16** and **20****),** as opposed to the insertion of a single strip **302** of material (as described above).

An anchor stent (not shown) could be utilized to temporarily secure the end of the cinching member **304** (or the end **308** of the loop **306)** inside the gastric lumen during the insertion procedure, For example, an anchor stent enclosing a portion of the cinching member **304** would be inserted into the pylorus and lodged therein. One end of the cinching member **304** (or loop **306)** enclosed within the anchor stent is then removed therefrom and pulled out of the subject, The other end of the cinching member **304** (or loop **306)** remains attached to the anchor stent. The intragastric device **300** can then be inserted into the gastric lumen by pushing or sliding the strip **302** (or bundles) down the cinching member **304** (or loop **306),** the end of which remains secured within the gastric lumen by the anchor stent. Once the insertion procedure is removed, then the anchor stent and any excess cinching member **304** are removed.

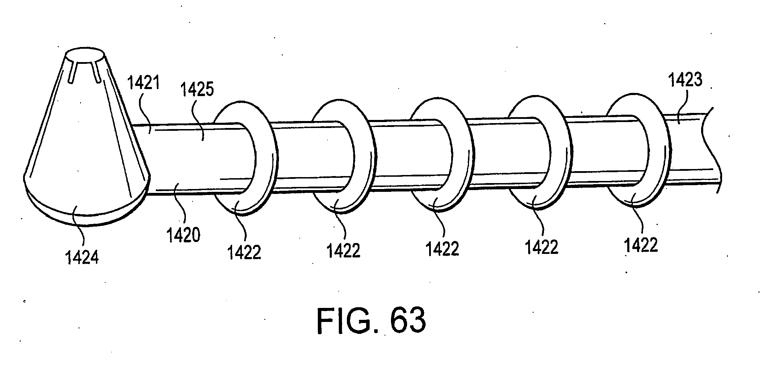

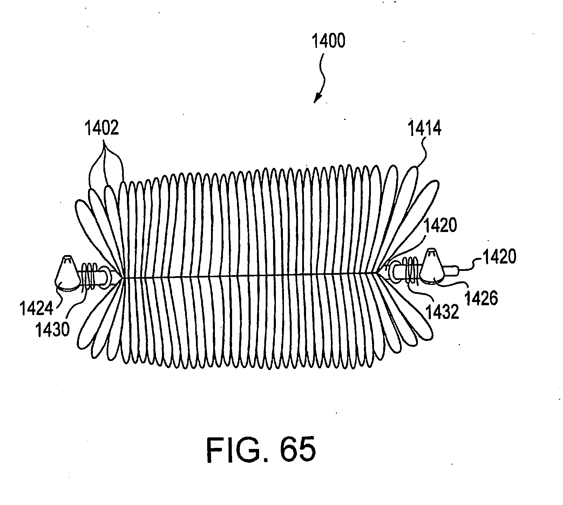

**FIGS. 63-65** depict yet another embodiment. In this embodiment, the intragastric member **1400** (see **FIG. 65****)** utilizes an elongate member **1420** to deliver and secure the intragastric member **1400** into the gastric lumen of the patient. The intragastric member **1400** is bundled by passing the elongate member **1420** through the aperture **1412** in the strip **1402** **FIG. 65****)** upon delivery into the gastric lumen of the patient. The elongate member **1420** can be attached to a distal end of a wire guide to facilitate delivery, as shown in **FIG. 18****.** The intragastric member **1400** is bundled onto the elongate member **1420** to deliver the intragastric member **1400** into the gastric lumen in stages and allow the loops to be pulled together to form a secured intragastric member **1400** upon delivery to the lumen of the patient.

In this embodiment, the elongate member **1420** comprises a plurality of ribs 1422 dispensed along the length of the elongate member **1420.** The ribs **1422** are spaced along the elongate member **1420** to engage the intragastric member **1400** after insertion into apertures **1412** of the intragastric member **1400.** The elongate member **1420** further comprises a distal stopper **1424,** such as a pawl, engaged to a distal end **1421** of the elongate member **1420** and a proximal stopper **1426,** such as a second pawl, attached to a proximal end **1423** of the elongate member **1420.** The ribs **1422** provide a ratcheting action upon engagement with a lumen **1419** of the proximal stopper **1426,** similar to a pawl mechanism. The ribs **1422** also allow the elongate member **1420** to be pulled only in a proximal direction upon engagement with the proximal stopper **1426.**

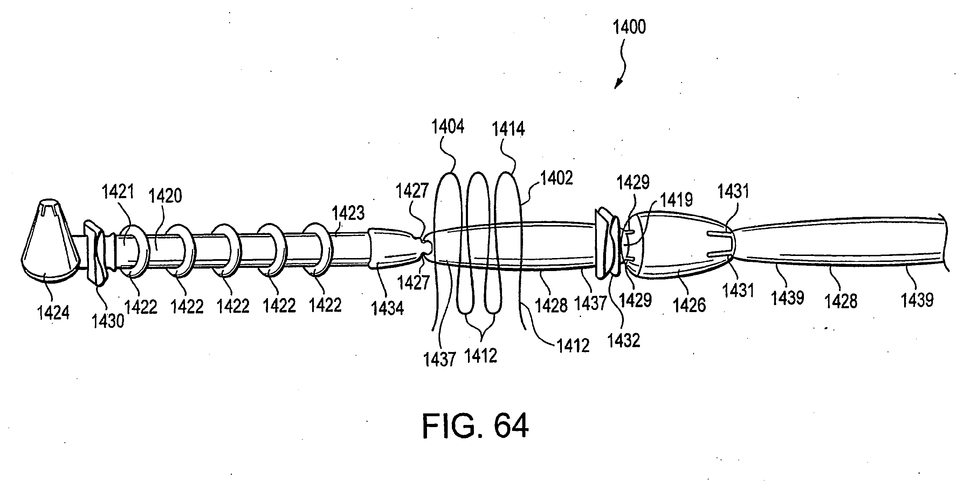

As best shown in **FIG. 64****,** the proximal stopper **1426** is engaged to the elongate member **1420** utilizing a cinching member **1428,** such as a nylon thread or similar thread-like structure. The cinching member **1428** comprises a proximal end **1439** and a distal end **1437.** The distal end **1437** of the cinching member **1428** is looped through a lumen **1427** located on a proximal end of a connector **1434** and passes through a lumen **1429** located on a distal end of the proximal stopper **1426.** The proximal end **1439** of the cinching member **1428** passes through a lumen **1431** located on the proximal end of the proximal stopper **1426** to facilitate delivery of the intragastric member **1400.** The proximal stopper **1426** slides along the cinching member **1428** upon delivery into the gastric lumen. The stoppers **1424, 1426** are utilized to compress the strip **1402** so as to form the intragastric member **1400** having a configuration similar to that shown in **FIG. 65****.**

**FIG. 65** depicts an embodiment of the intragastric member **1400** in a bundled, final configuration after delivery or insertion into the gastric lumen. Accordingly, the intragastric member **1400** is preferably inserted into the gastric lumen in stages. For example, and as shown in FIG. **64****,** the intragastric member **1400** is separated into a plurality of separate bundles **1414** to improve the handling of the material and prevent the strip **1402** from becoming tangled or contaminated. The separate bundles **1414** of the intragastric member **1400** are delivered to the elongate member **1420** one at a time after being inserted into the gastric lumen. Before delivery, a second distal stopper **1430,** such as a drag, is engaged to the distal end **1421** of the elongate member **1420** between the distal stopper **1424** and the first rib **1422.** A second proximal stopper **1432,** such as a second drag, is connected to the proximal end **1439** of the cinching member **1428** between the connector **1434** and proximal stopper **1426** after delivery of all the bundles **1414** to the lumen. Both the second distal stopper **1430** and the second proximal stopper **1432** are sized and configured so as to not pass over the stoppers **1424,1426** or through the apertures **1412** in the strip **1402.**

Once a distal end **1421** of the elongate member **1420** is positioned in the gastric lumen, the first bundle **1414** is threaded over the proximal (rearward) end by passing the apertures **1412** over the elongate member **1420.** A pusher (not shown) is then positioned over the proximal end of the elongate member **1420,** and slid towards the distal end **1421** of the elongate member **1420** so as to push the folds **1404** of the first bundle against the second distal stopper **1430** and the distal stopper **1424.** This procedure is then repeated by threading subsequent bundles **1414** over the elongate member **1420** and pushing them against the previously inserted bundles **1414** until a11 of the bundles **1414** have been inserted into the gastric lumen (see FIG. **65****).** The bundles **1414** are then secured together by pushing the proximal stopper **1426** distally to fasten the bundles **1414.** The proximal stopper **1426** is passed along the ribs **1422** of the elongate member **1420** providing tactile confirmation that the proximal stopper **1426** is securely fastened. The cinching member **1428** is subsequently removed and the delivery device, such as the wire guide, is then withdrawn so as to leave the intragastric member **1400** secured along the elongate member **1420.** To remove the intragastric member **1420** from the gastric lumen, the elongate member **1420** is typically cut so as to release the folds **1404.** One end of the strip **1402** is then grasped by an endoscopic or similar device and pulled out of the patient.

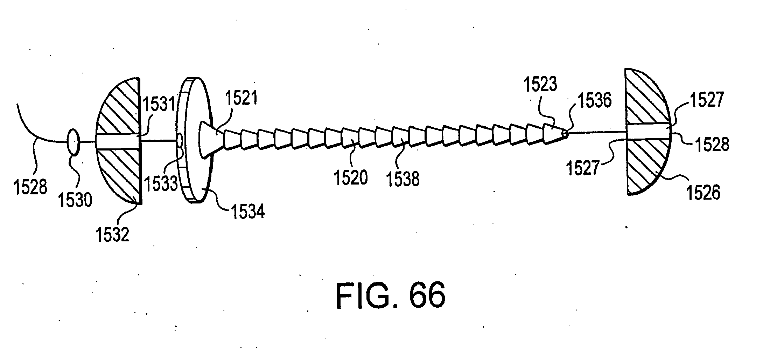

**FIGS. 66-68** depict another embodiment. In this embodiment, an elongate member **1520** is used to deliver the intragastric member **1400** into the gastric lumen of the patient. In this embodiment, the elongate member **1520** includes a plurality of ribs **1538** engaged between a proximal end **1523** and a distal end **1521** of the elongate member **1520.** The elongate member **1520** further comprises a lumen **1536** configured to receive a cinching member **1528,** such as a nylon thread or similar thread-like structure, to facilitate delivery of the intragastric member **1400.**

The elongate member **1520** further comprises a distal stopper **1532,** such as a button, engaged to the distal end **1521** of the elongate member **1520** and a proximal stopper **1526,** such as a second button, engaged to a proximal end **1523** of the elongate member **1520.** The proximal stopper **1526** includes a lumen **1527** configured to receive the cinching member **1528** that passes through the lumen **1536** of the elongate member **1520.** The distal end **1521** of the elongate member **1520** also includes a locking member **1534** that engages a portion of the intragastric member **1400** after complete delivery of the intragastric member **1400.** The distal stopper **1532** and the locking member **1534** engage the strip **1402** of the intragastric member **1400** and trap the intragastric member **1400** between the distal stopper **1532** and the locking member **1534** to secure the intragastric member **1400** upon delivery into the gastric lumen. Both the distal stopper **1532** and the locking member **1534** include a lumen **1531, 1533** configured to receive the cinching member **1528** that passes through the lumen **1536** of the elongate member **1520.** In addition, a secondary distal stopper **1530** is engaged along the cinching member **1528** adjacent the distal end of the distal stopper **1532** to further secure the intragastric member **1400** upon delivery.

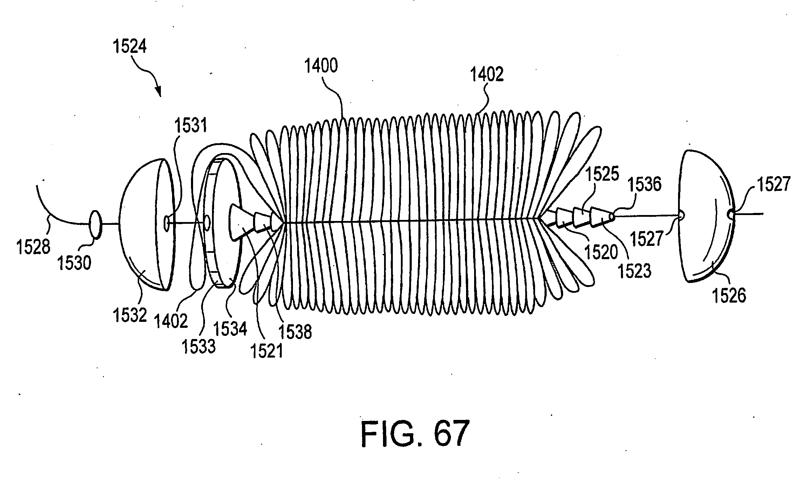

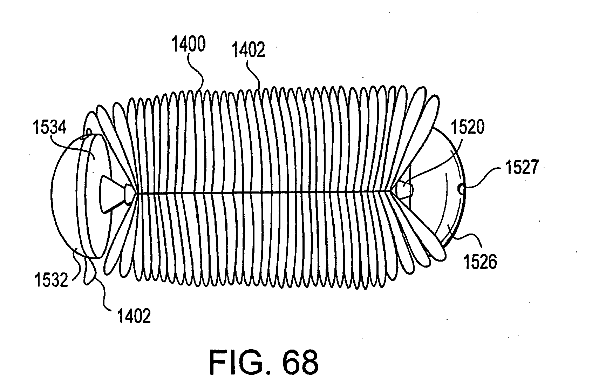

**FIG. 67** depicts the elongate member **1520** after delivery of the intragastric member **1400** into the gastric lumen. In this embodiment, the intragastric member **1400** is deployed by extending the strip **1402** along the elongate member **1520** by pushing the intragastric member **1400** over the elongate member **1520,** so as to secure the intragastric member **1400** along the distal stopper **1532** and the elongate member **1520.** The elongate member **1520** is then secured and closed by engaging the ribs **1538** of the elongate member **1520** to the lumen **1527** of the proximal stopper **1526.** Ribs **1538** are included along the elongate member **1520** to provide a ratcheting action upon engagement with the proximal stopper **1526.** The ribs allow the elongate member **1520** to pass only in a forward direction upon engagement and prevent the elongate member **1520** from moving in an opposite direction after engagement with the lumen **1527** of the proximal stopper **1526.** As the elongate member **1520** is closed, the strip **1402** is compressed so as to form the intragastric member **1400** having a configuration similar to that shown in **FIGS. 67** and **68****.** After the intragastric member **1400** has been secured inside the gastric lumen, then the portion of the elongate member **1520** extending beyond the proximal stopper **1526** can be severed with an endoscopic scissors and removed. In addition, the intragastric member **1400** can be inserted in bundles, as opposed to the insertion of a single strip **1402** of material (as described above).

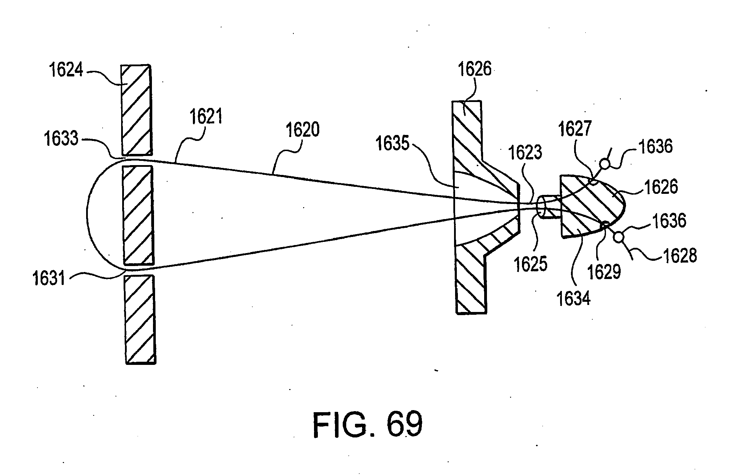

**FIG. 69** depicts yet another embodiment. In this embodiment, a cinching member **1620** is used to deliver the intragastric member **1400** into the gastric lumen of the patient. The cinching member **1620** includes a distal stopper **1624,** such as a button, and a proximal stopper **1626,** such as a second button, wherein the distal stopper **1624** is engaged to a distal end **1621** of the cinching member **1620** and the proximal stopper **1626** is engaged to a proximal end **1623** of the cinching member **1620.** The cinching member **1620** is engaged to the distal stopper **1624** by looping the cinching member **1620** through a first lumen **1631** and a second lumen **1633** of the distal stopper **1624.**

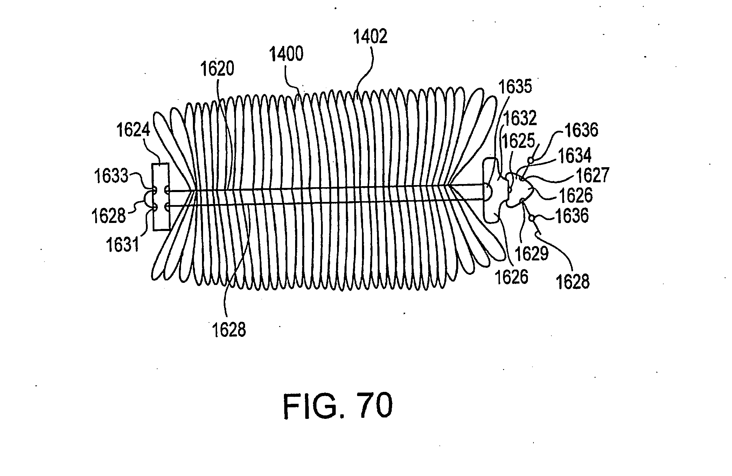

As shown in **FIG. 69****,** a secondary proximal stopper **1634** is engaged along the cinching member **1620** adjacent the proximal end of the proximal stopper **1626** to further secure the intragastric member **1400** upon delivery. The proximal stopper **1626** comprises a lumen **1635** configured to receive the cinching member **1620** as it passes from the distal stopper **1624.** The lumen **1635** further operates as a point of connection to interlock the secondary proximal stopper **1634** to the proximal stopper **1626** after the stoppers **1634, 1626** are fastened after complete delivery of the intragastric member **1400** (FIG. **70****).** The secondary proximal stopper **1634** further comprises a first lumen **1625,** a second lumen **1627** and a third lumen **1629,** wherein the first lumen **1625** receives the cinching member **1620** as it is passes from the lumen **1635** of the proximal stopper **1626,** and wherein the second lumen **1627** and the third lumen **1629** receive the cinching member **1620** as it exits the first lumen **1625** of the second portion **1634.**

**FIG. 70** depicts the cinching member **1620** after delivery of the intragastric member **1400** in a bundled, final configuration. In this embodiment, the intragastric member **1400** is deployed by extending the strip **1402** along the cinching member **1620** after the intragastric member **1400** has been delivered into the gastric lumen and the intragastric member **1400** is pushed over the cinching member **1620,** so as to secure the intragastric member **1400** along the cinching member **1620.** The proximal stopper **1626** and second proximal stopper **1634** are closed by engaging a distal end of the second proximal stopper **1634** to the lumen **1635** of the proximal stopper **1632** and passing the proximal end **1623** of the cinching member **1620** through the second lumen **1627** and third lumen **1625** of the second proximal stopper **1634.** After the cinching member **1620** is closed, the strip **1402** is compressed so as to form the intragastric member **1400** having a bundled, final configuration. The ends of the cinching member **1620** can be tied in knots **1636** to further secure the intragastric member **1400** in the gastric lumen. After the intragastric member **1400** has been secured inside the gastric lumen, then the portion of the cinching member **1620** extending beyond the second proximal stopper **1634** can be severed with an endoscopic scissors and removed. In addition, the intragastric member **1400** can be inserted in bundles, as opposed to the insertion of a single strip **1402** of material (as described above).

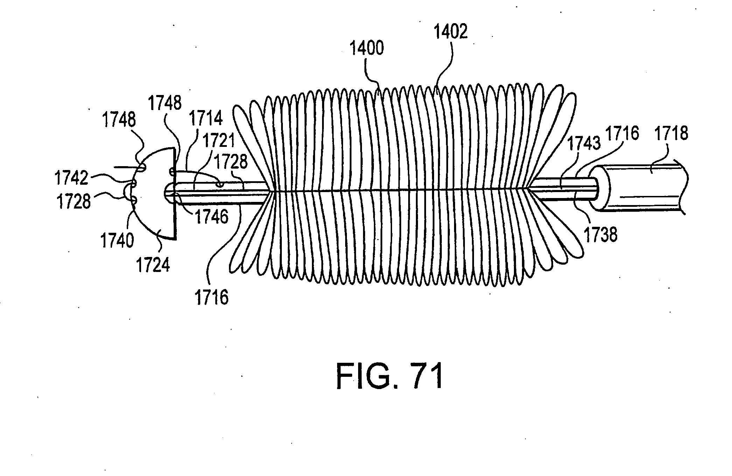

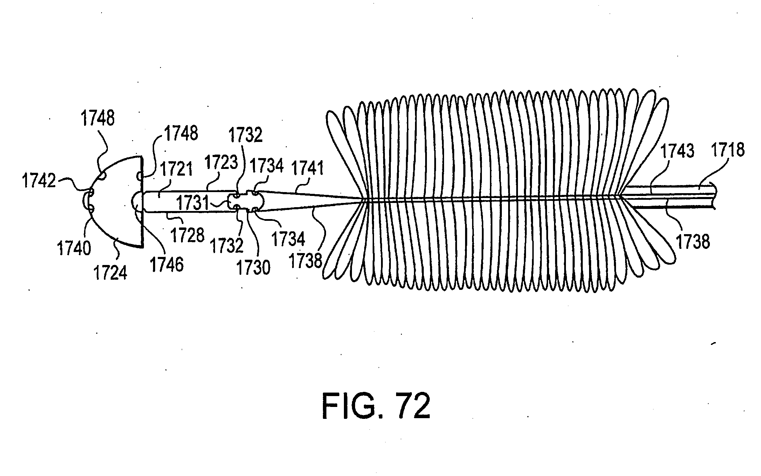

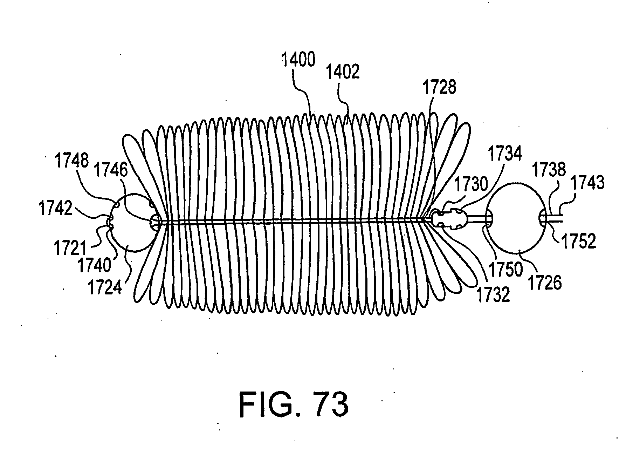

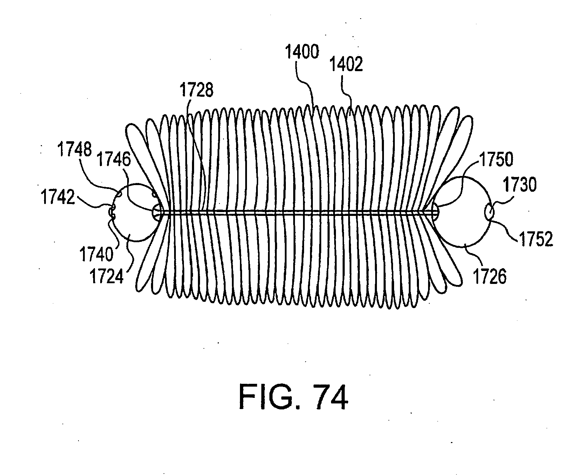

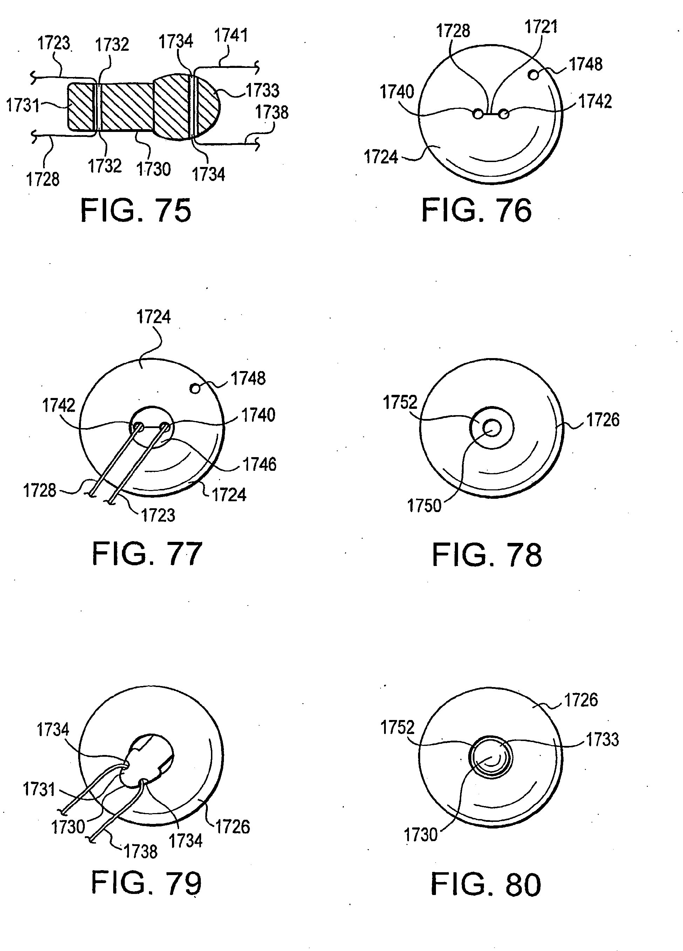

**FIGS. 71-80** depict yet another embodiment of the intragastric member **1400.** In this embodiment, the intragastric member **1400** utilizes a first cinching member **1728** and a second cinching member **1738,** such as a nylon thread or similar thread-like structure, to deliver the intragastric member **1400** into the gastric lumen of the patient. Similar to the above embodiments, the intragastric member **1400** may be loaded and delivered to the first cinching member **1728** using a wire guide **1714,** an inner delivery tube **1716** and an outer delivery tube **1718,** or other similar delivery devices **(****FIG. 71****).** As shown in **FIG. 72****,** the first cinching member **1728** comprises a proximal end **1723** and a distal end **1721** wherein the intragastric member **1400** is threaded between the proximal end **1723** and the distal end **1721** of the first cinching member **1728.** A distal stopper **1724,** such as a button, is disposed on the distal end **1721** of the first cinching member **1728** for securing the intragastric member **1400** along the first cinching member 1728 **(****FIG.** 73). A proximal stopper **1726,** such as a second button, is disposed on the proximal end **1723** of the first cinching member **1728** for locking the intragastric member **1400** along the first cinching member **1728 (****FIG.** 74). The first cinching member **1728** is further engaged to a distal end **1731** of a second proximal stopper **1730,** such as a bead, and the second cinching member **1738** is **engaged** to a proximal end **1733** of the second proximal stopper **1730 (****FIG. 75****).**

As illustrated in **FIGS. 76-77****,** the distal stopper **1724** is engaged to the first cinching member **1728** by looping the first cinching member **1728** through a first lumen **1732** of the second proximal stopper **1730.** The distal stopper **1724** comprises a first lumen **1746,** a second lumen **1740** and a third lumen **1742,** wherein the first cinching member **1728** enters the first lumen **1746 and** loops through the second lumen **1740** and third lumen **1742** before exiting the first lumen **1746** to form a complete loop with the first lumen **1732** of the second proximal stopper **1730.** The first lumen **1732** also provides an opening to secure the distal stopper **1724** to the inner delivery tube **1716** during delivery. In addition, the distal stopper **1724** comprises a fourth lumen **1748** for securing the wire guide **1714** during delivery.

As illustrated in **FIGS. 78-80****,** the proximal stopper **1726** is engaged to the second cinching member **1738** by looping the second cinching member **1738** through a second lumen **1734** of the second proximal stopper **1730.** The proximal stopper **1726** comprises a first lumen **1750** and a second lumen **1752** for receiving the second proximal stopper **1730.** The first lumen **1750** comprises a first diameter and the second lumen **1752** comprises a second diameter wherein the first diameter is smaller than the second diameter. The first diameter is configured to allow the second proximal stopper **1730** to only pass in a proximal direction through the first lumen **1750** of the proximal stopper **1726 (****FIG. 78****),** and prevents the second proximal stopper **1730** from passing distally back through the first lumen **1750 (****FIG. 79****).** Therefore, once the second proximal stopper **1730** passes through the first lumen **1750** it is secured in the first lumen **1750 (****FIGS. 78-80****).**

**FIG. 74** depicts the first cinching member **1728** after delivery of the intragastric member **1400** in a bundled, final configuration. In this embodiment, the intragastric member **1400** is deployed by extending the strip **1402** along the first cinching member **1728** after the intragastric member **1400** has been delivered into the gastric lumen and the intragastric member 1400 is pushed over the first cinching member **1728,** so as to secure the intragastric member **1400** along the first cinching member **1728.** The distal stopper **1724** and the proximal stopper **1726** are closed by pulling the second cinching member **1738** proximally until the second proximal stopper **1730** fully engages the first lumen **1750** of the proximal stopper **1726.** After the first cinching member **1728** is closed, the strip **1402** is compressed so as to form the intragastric member **1400** having a bundled, final configuration. The second cinching member **1738** is then pulled until it is removed from the intragastric member **1400.**

Experimental testing of the present invention has been conducted on mammals. In particular, an embodiment of an intragastric member similar to the embodiment shown in **FIGS. 19-21** and **FIGS. 64-74** was inserted into the gastric lumens of a group of ten (10) pigs for a period of 49 days. No deaths or major complications were observed in any of the test subjects. The initial weight for the test subjects was measured to be in the range of 25.0 to 31.2 kg, with an average weight of 27.8 kg. At the end of the 49 day testing period, the weight of the test subjects was measured to be in the range of 29.5 to 39.0 kg, with an average weight of 34.5 kg. The anticipated weight for the test subjects at the end of the testing period, in view of the normal and expected growth for these animals, was 57 kg. Accordingly, the test subjects gained an average weight that was significantly less than the weight gain observed in similar animals without the intragastric member.

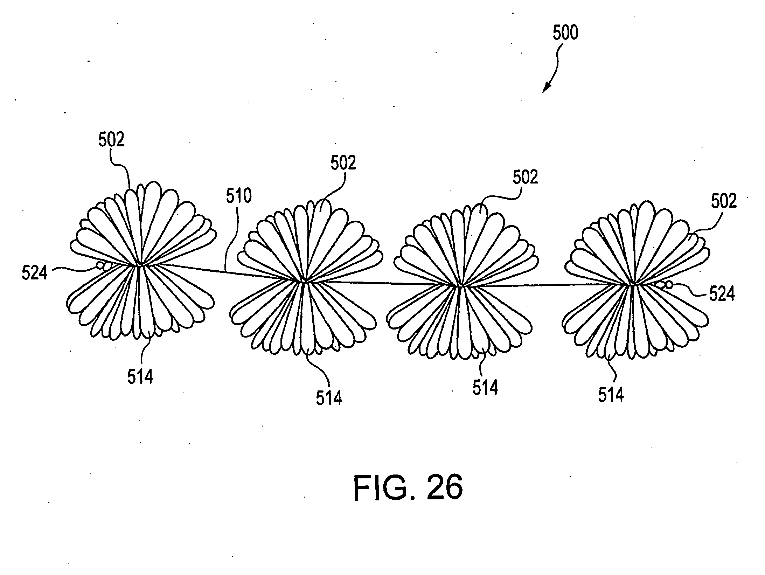

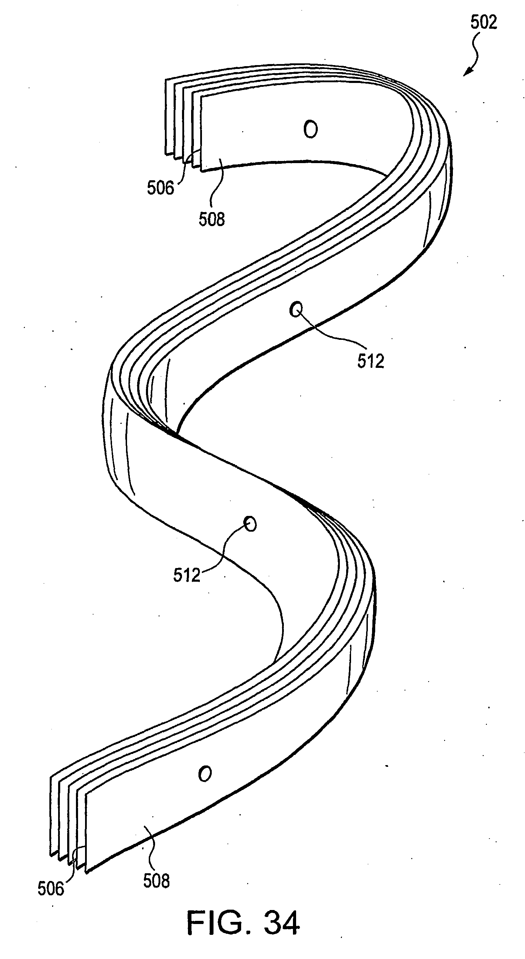

**FIG. 26** depicts another embodiment of an intragastric member **500.** In this embodiment, the intragastric member **500** comprises a plurality of strips of high-density polyethylene **502** that have been folded and bundled in the general shape of a butterfly or a bowtie. As best seen in **FIG. 34****,** each strip of high-density polyethylene **502** of the embodiment is formed from a tube of material having a wall thickness of 40-50 microns and a perimeter of 3 cm. The material is then stacked and folded to form a plurality of strips **502** having two walls **506,508,** wherein each wall **506,508** has a width of 1.5 cm. Of course, the strip **502** could comprise a different number of walls **506,508,** have a different width and thickness, or be formed from a tube of material.

In the embodiment shown in **FIG. 26****,** the intragastric member **500** is formed from approximately five individually stacked strips **502.** The intragastric member **500** is bundled by passing a cinching member **510,** such as a nylon thread or similar thread-like structure, through an aperture **512** in the strip **502** at the center of the each bundle **514.** As best seen in **FIG. 34****,** the apertures **512** are formed in each wall **506,508** of the strip **502,** and are spaced so that bundles **514** are formed 20 cm in length when adjacent apertures **512** are pulled together to form the intragastric member **500** shown in **FIG. 26****.** In other words, the apertures **512** are located every 20 cm along the length of the strip **502.** Alternate embodiments of an intragastric member may include varying numbers of strips of high-density polyethylene having longer or shorter lengths depending on the desired size and mass of the intragastric member.

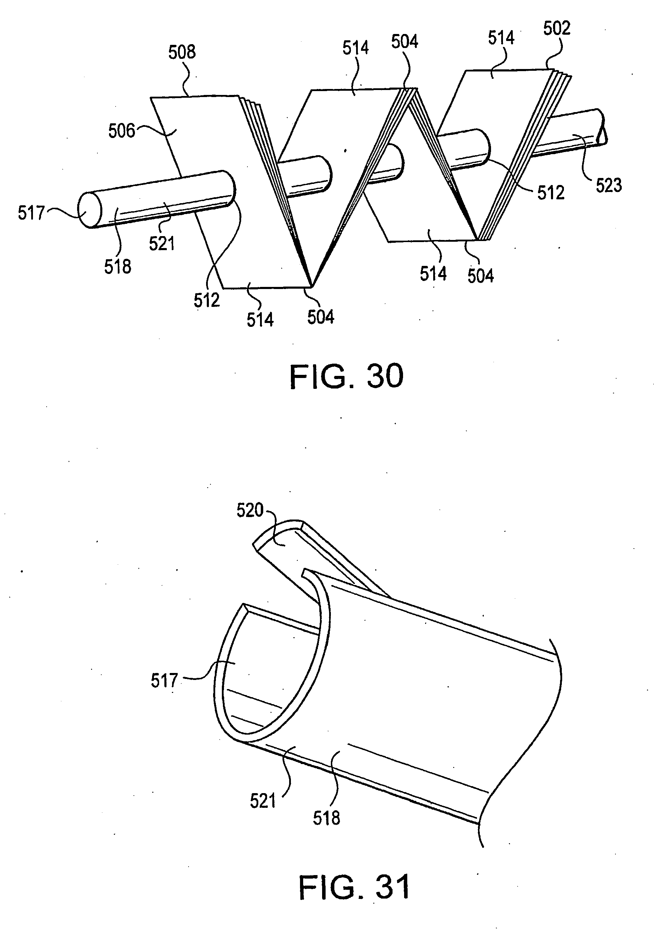

The embodiment of the intragastric member **500** shown in **FIG. 26** may be too large for delivery or insertion into the gastric lumen while in its bundled, final configuration. Accordingly, the intragastric member **500** is preferably inserted into the gastric lumen in stages. For example, and as shown in **FIG. 30****,** the intragastric member **500** is separated into four (4) separate bundles **514,** each of which comprise approximately five (5) layers of the high-density polyethylene strips **502.** The stacked layers of strips **502** provide the bundle **514** with an increased volume, thereby providing a thicker intragastric member **500.**

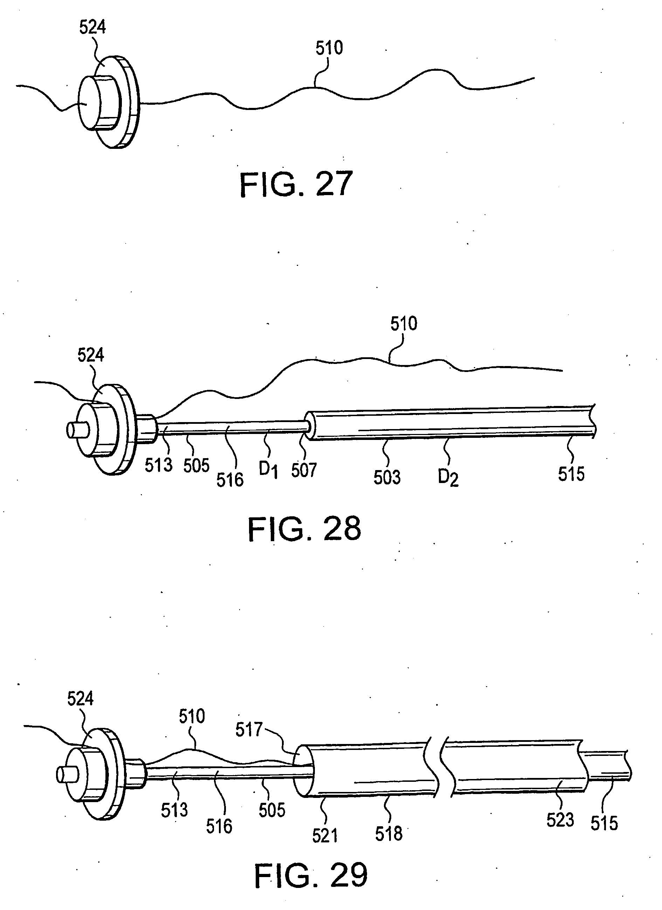

As shown in **FIGS. 27-33****,** the separate bundles **514** of the intragastric member **500** are inserted into the gastric lumen one at a time by loading the intragastric member **500** over an outer delivery tube **518** and delivering through the esophagus via an overtube into the gastric lumen where the strips **502** are gathered in a butterfly or bow-tie formation. The intragastric member **500** is loaded onto the outer delivery tube **518** by threading the outer delivery tube **518** through the apertures **512** of each strip **502** (see **FIG. 30****).** The outer delivery tube **518** comprises a proximal end **523,** a distal end **521,** and a main opening **517** through which an inner delivery tube **516** passes (see **FIG. 29****).** The inner delivery tube **516** comprises a proximal end **515** and a distal end **513,** wherein the distal end **513** is engaged with at least one stopper **524** (see **FIG. 28****).** The stopper **524** is further engaged with the cinching member **510** which passes through the main lumen **517** of the outer delivery tube **518.** Delivery of the intragastric member **500** begins by inserting the distal end **513** of the inner delivery tube **516** in the gastric lumen of the patient while the proximal end **515** will remain external to the patient.

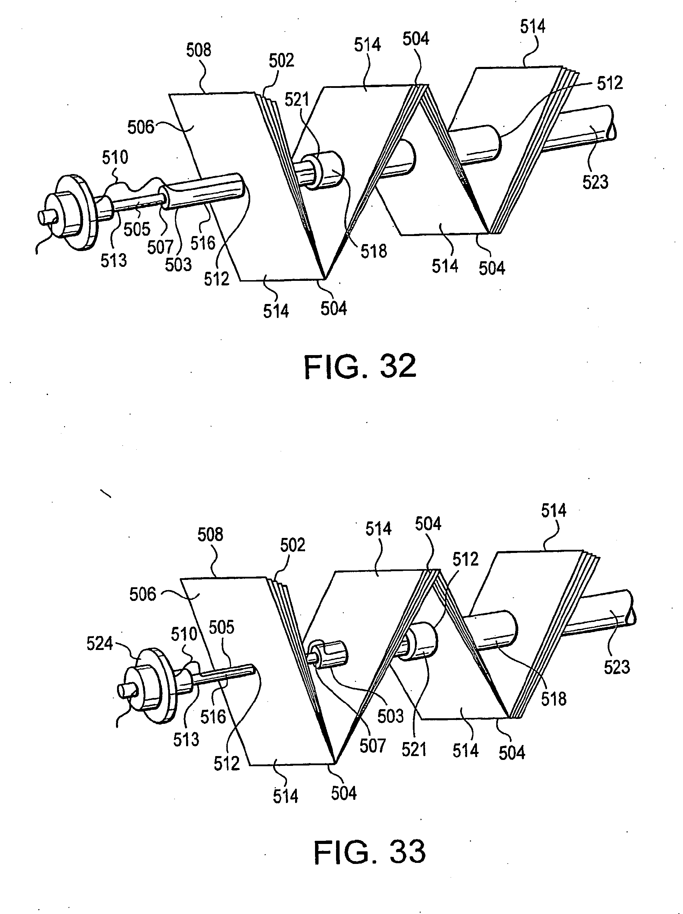

Once the distal end **513** of the inner delivery tube **516** is positioned in the gastric lumen, the first bundle **514** is threaded over the proximal (rearward) end **523** of the outer delivery tube **518** by passing the apertures **512** over the outer delivery tube **518.** Each bundle **514** is manually pushed from the proximal end **523** of the outer delivery tube **518,** and slid towards the distal end **513** of the inner delivery tube **516** so as to push the folds **504** of the first bundle **514** past the distal end **521** of the outer delivery tube **518** (see **FIGS. 32-33****).** The outer delivery tube **518** comprises a delivery tip **520** that facilitates the one way delivery of each bundle **514** onto the inner delivery tube **516** (see **FIG. 31****).**

The inner delivery tube **516** further comprises a proximal section **503,** a distal section **505,** and a transitional section **507,** wherein the diameter D1 of the distal section **505** may be smaller than the diameter D2 of the proximal section **503** (see **FIG. 28****).** As shown in **FIGS. 32-33****,** the diameter of the proximal section 503 of the inner delivery tube **516** is sized and configured to align with the apertures **512** of the strips **502** after each bundle **514** transitions from the outer delivery tube **518** onto the inner delivery tube **516** during delivery. The plurality of stacked strips **502** further allow the apertures **512** to remain frictionally engaged with the proximal section **503** of the inner delivery tube **516** during delivery. As the outer delivery tube **518** is withdrawn, the subsequent bundle **514** is pushed past the transitional section **507** of the inner delivery tube **516** onto the distal section **505.** Each bundle **514** is generally retained in the distal section **505** of the inner delivery tube **516,** wherein the length of the distal section **505** is approximately equivalent to the length of the bundle **514**. The varying diameter of the inner delivery tube **516** combined with the stacked strips **502** allow the bundles **514** to be easily delivered. A skilled artisan would appreciate that other designs comprising an inner delivery tube of different diameters could be utilized.

This procedure is then repeated by threading subsequent bundles **514** over the outer delivery tube **518** and pushing them against the previously inserted bundles **514** until all of the bundles **514** have been inserted into the gastric lumen (see **FIG. 33****).** The bundles **514** are then secured together by pushing a proximal stopper or similar device (see **FIG. 26****)** along the outer delivery tube **518** so as to press against the last bundle **514** to be inserted. The outer delivery tube **518** is then withdrawn so as to leave the cinching member **510** extending through the apertures **512** of all of the bundles **514.** The cinching member **510** is then tied or otherwise secured to the proximal stopper so as to form a complete intragastric member **500** as shown in **FIG. 26****.** For example, in an alternate embodiment, the stopper can be secured by crimping together a first end and second end of the cinching member **510** with a metal band, such as a ferrule.

To remove the intragastric member **500** from the gastric lumen, the cinching member **510** is typically cut so as to release the folds **504.** One end of the strip **502** is then grasped by an endoscopic or similar device and pulled out of the patient.

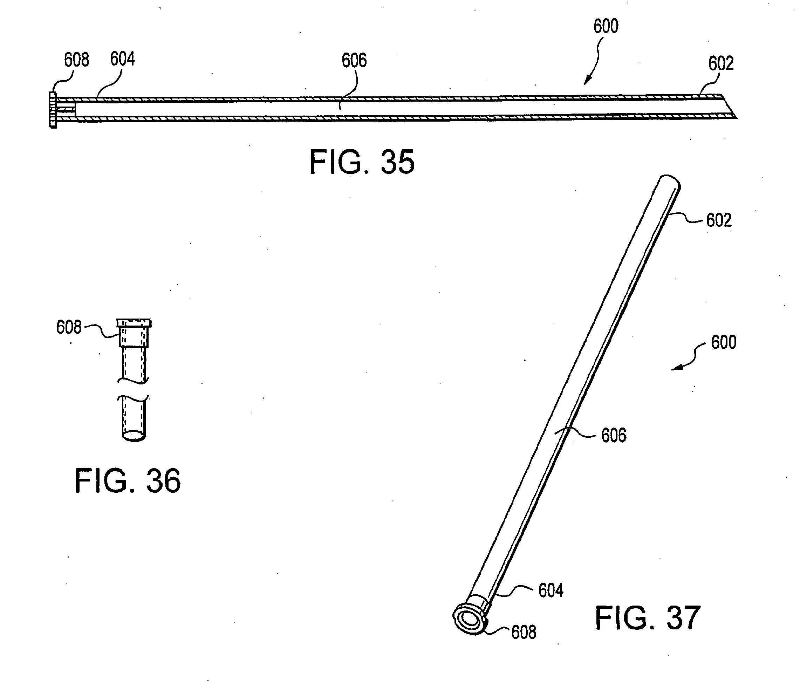

**FIG. 35** depicts an overtube **600** that is used to deliver an intragastric member **500** to the gastric lumen of the patient. The overtube **600** is used in combination with an endoscope to establish a passageway to a target delivery site in the stomach. Once the overtube **600** is positioned in the gastric lumen of the patient, the intragastric member **500** is passed through the overtube **600,** and is used to deliver the intragastric member **500** to the gastric lumen. Once the desired delivery in the gastric lumen is complete, the overtube **600** is removed.

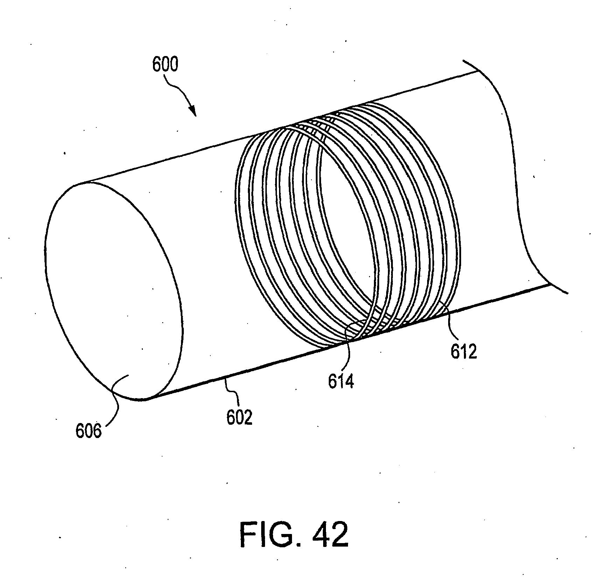

The overtube **600** comprises a proximal end **604,** a distal end **602,** and a main lumen **606.** Any arrangement of the main lumen **606** is contemplated. **FIGS. 35** and **37** illustrate an embodiment of the overtube **600** wherein the overtube **600** is flexible and includes a single-piece construction. Alternatively, several tubes may be bonded together to form the flexible overtube **600** (see **FIG. 36****).** The overtube **600** can be made from any suitable material known in the art including, but not limited to, polyethylene ether ketone (PEEK), polytetrafluorethylene (PTFE), polyamide, polyurethane, polyethylene and nylon, including multi-layer or single layer structures and may also include reinforcement wires, braid wires, coils and or filaments. In the embodiment of the overtube **600** shown in **FIG. 42****,** the overtube **600** comprises a reinforcement member **612,** such as metal coil **614,** molded into the main lumen **606** of the overtube **600.** The reinforcement member **612** provides structural reinforcement to the overtube **600,** thereby decreasing the minimum radius that the overtube **600** can withstand without kinking or otherwise reducing the inside diameter of the overtube **600.**

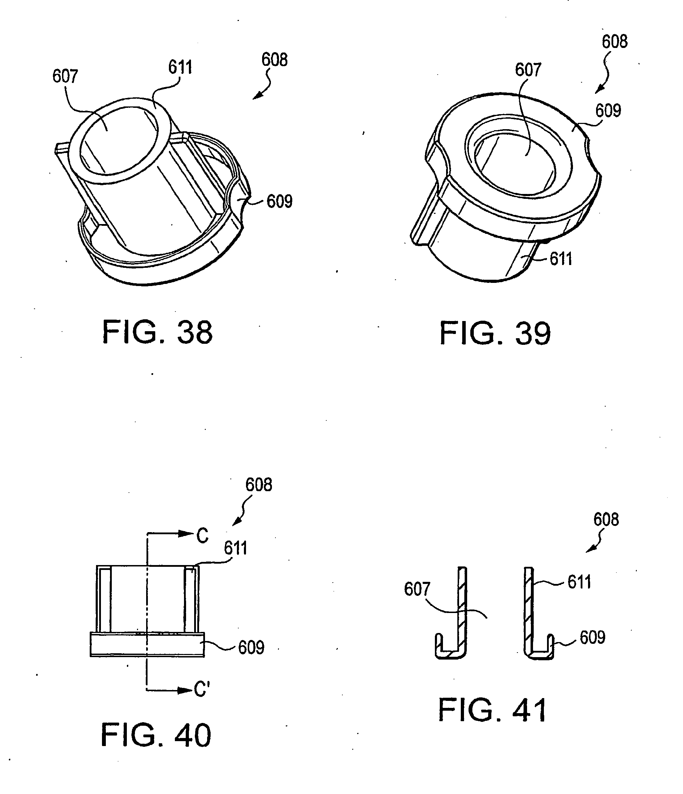

Optionally, the overtube **600** further comprises a valve **608** over its proximal end **604,** as illustrated in **FIGS. 35-41****.** The valve **608** forms a seal between the flexible overtube 600 and any secondary device, such as an endoscope or the like, that is advanced therethrough to prevent the loss of any fluid that is introduced through the endoscope or other device. In one embodiment, the valve **608** is removable. The valve **608** has a proximal end **609** and a distal end **611** (see **FIGS. 38-39****),** wherein the distal end **611** is engaged with the proximal end **604** of the overtube 600 (see **FIG. 35****).** The valve **608** further includes a lumen **607** which provides an opening for passing a secondary device (see **FIG. 38****),** such as an endoscope or intragastric member **500.** The valve **608** also provides a connection point for engaging any suitable secondary device used in connection with the overtube **600.** One of ordinary skill in the art would know how to assemble valve **608** over the proximal end **604** of overtube **600.**

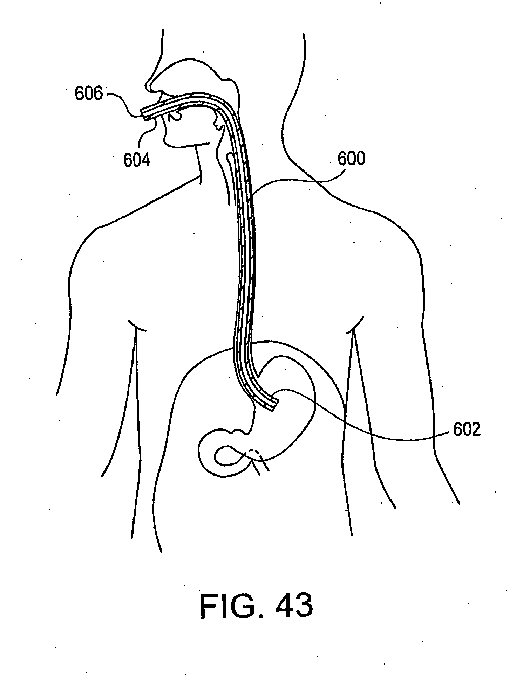

The main lumen **606** is configured to receive and pass an intragastric member **500,** or suitable secondary device, such as an endoscope (see **FIG. 43****).** The main lumen **606** ranges in size depending on the size of the intragastric member **500** deployed. The size of the overtube 600 and corresponding intragastric member **500** are provided for illustrative purposes only and are not intended to be construed as a limitation of the present invention. As one of ordinary skill in the art would appreciate, since the intragastric member **500** and the endoscope and are advanced through the main lumen **606,** the size of the main lumen **606** is related to the size of either the intragastric member **500** or the endoscope, which ever is larger. One of ordinary skill in the art would also appreciate that the size of the intragastric member **500** is related to the length, width, and number of polyethylene strips comprising the intragastric member **500.** Thus, a flexible overtube **600** may have smaller or larger dimensions depending on the size of the intragastric member **500,** endoscope or other secondary device used in conjunction with the overtube **600** and therefore any overtube **600** of varying dimensions is contemplated as being within the scope of the claims of the present invention.

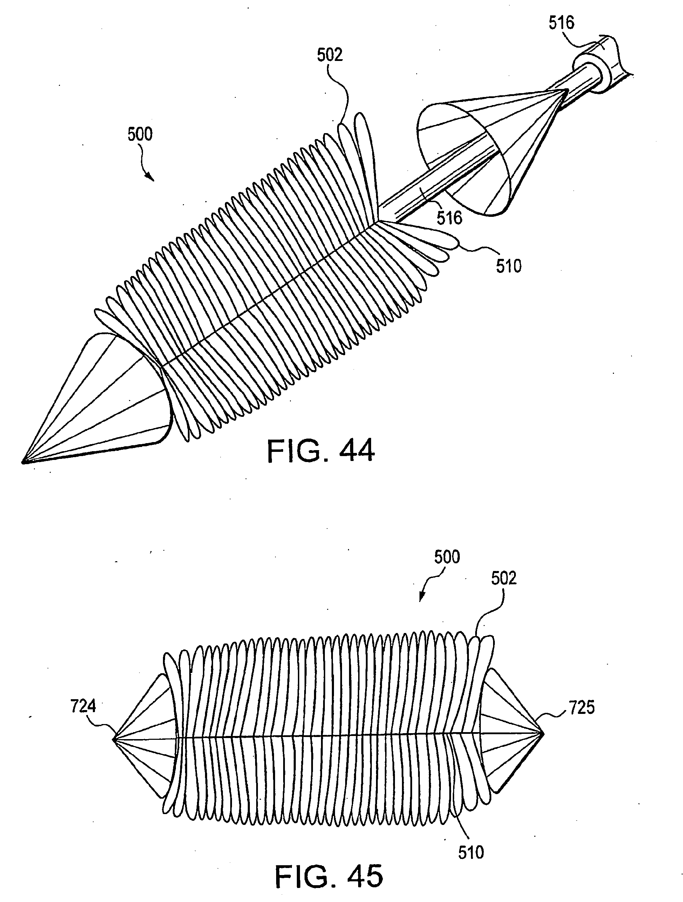

**FIGS. 44-45** depict another embodiment wherein the intragastric member **500** comprises a distal stopper **724** engaged with a distal end of the intragastric member **500** and a proximal stopper **725** engaged with a proximal end of the intragastric member **500** to further secure the intragastric member **500** in its intended formation. In this embodiment, the distal stopper **724** and the proximal stopper **725** comprise a pair of preformed expandable stoppers having an umbrella-like shape.

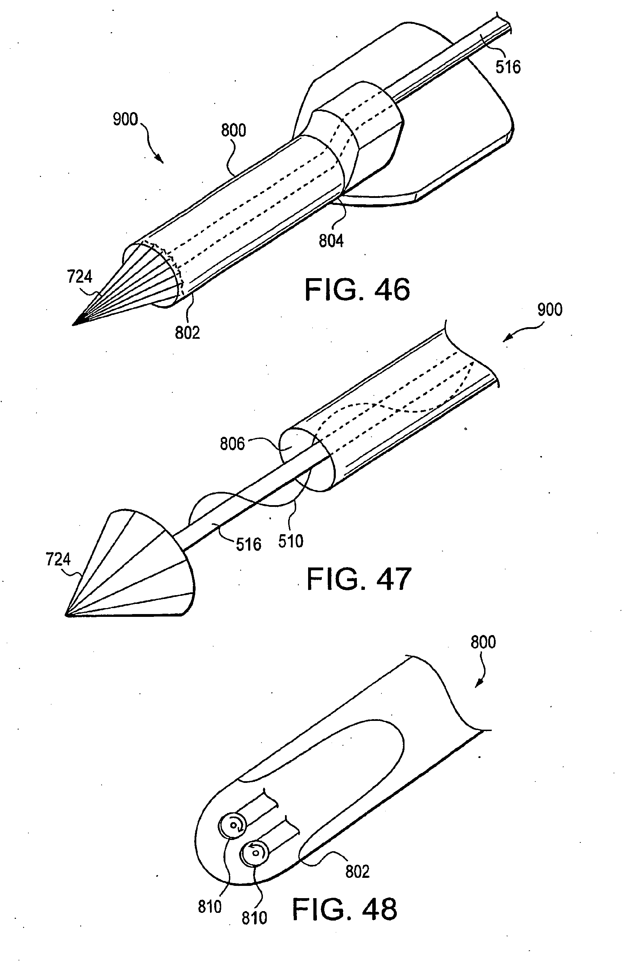

During delivery, the distal stopper **724** is engaged with an end of the inner delivery tube **516** and cinching member **510** while in an unexpanded first position (see **FIG. 46****).** The distal stopper **724** passes through the main lumen of an overtube during delivery and upon delivery of the intragastric member **500** into the gastric lumen, the distal stopper **724** expands to a second position wherein the diameter of the distal stopper **724** is suitable to retain the corresponding intragastric member **500** in its intended formation. Upon complete delivery of the intragastric member **500** into the gastric lumen, the proximal stopper **725** is engaged with a proximal end of the intragastric member **500** to further secure the intragastric member **500** in its intended formation.

The cinching member **510** is tied or otherwise secured to the distal stopper **724** and the proximal stopper **725** so as to form a complete intragastric member **500** as shown in **FIG. 45****.** The size and diameter of the distal stopper **724** and the proximal stopper **725** can vary depending on the size of the intragastric member **500.** The embodiment illustrated in **FIG. 45** includes two stoppers **724, 725** to secure the intragastric member **500** in its formation. However, alternative embodiments can include varying configurations. For example, one configuration can include a distal stopper 724 engaged to a distal end of the intragastric member **500** and a generic rubber stopper or other similar securing device engaged to a proximal end of the intragastric member **500.**

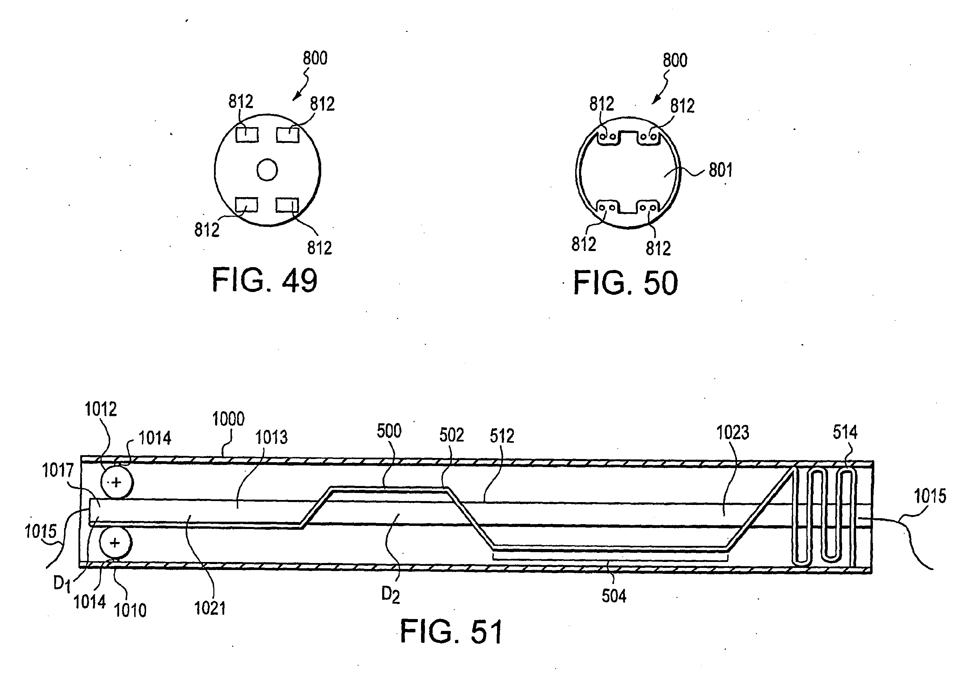

**FIGS. 46-47** depict a delivery system **900** in which the intragastric member **500** is delivered through an overtube **800** having a plurality of rollers **810** positioned along the lumen **806** of the overtube **800.** The rollers **810** can be positioned along at least one end of the overtube **800.** In this embodiment, a set of rollers **810** are positioned along a distal end **802** of the overtube **800** (see PIG. **48).** In addition, rollers **810** can be positioned on a proximal end **804** of the overtube **800.** The rollers **810** can rotate in a clockwise or counter clockwise direction to facilitate the intragastric member **500** as it passes along the lumen **806** during delivery. The overtube **800** can further include a plurality of grooves **812** along the lumen **806** of the overtube **800** (see **FIGS. 49-50****).** In the illustrative embodiments, four grooves **812** (see **FIG. 49****)** are located along the inside of the overtube **800,** however, a skilled artisan would appreciate that other designs could include a smaller or larger number of grooves **812.** Each groove **812** can be engaged with an end of the roller **810** that extends along the lumen **806** of the overtube **800.** Of course, the overtube **800** could comprise a different number of rollers **810,** or the rollers **810** could be eliminated entirely. The grooves **812** can also provide housing for a cable system used to pass the intragastric member **500** along the lumen **806** of the overtube **800.**

After the intragastric member **500** is loaded into the overtube **800,** the groove **812** and roller **810** assembly operate in conjunction to pass the intragastric member **500** from the proximal end **804** to the distal end **802** of the overtube **800.** Once the intragastric member **500** has been introduced into the gastric lumen, the cinching member **510** is pulled back through the overtube **800,** thereby releasing the intragastric member **500** into the gastric lumen.

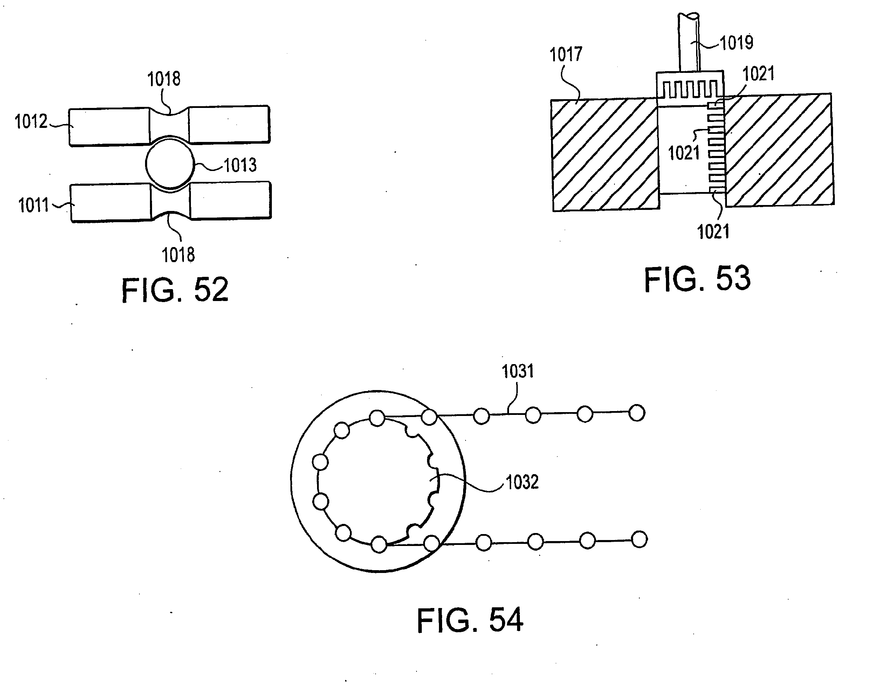

**FIG. 51** depicts another embodiment of a delivery system. In this embodiment, the separate bundles **514** of the intragastric member **500** are inserted into the gastric lumen one at a time by loading the intragastric member **500** into an overtube **1000** comprising mechanically driven rollers **1010,1012** and delivering it through the esophagus and into the gastric lumen where the strips **502** are gathered in a butterfly or bow-tie formation. The rollers **1010, 1012** are attached to the end of the overtube **1000** utilizing a connection mechanism such as brackets **1014,** posts or other suitable method within the scope of the invention. In addition, the rollers **1010, 1012** are connectable to an electric motor or similar means of power to cause and control rotation of the rollers **1010,1012** to facilitate delivery of the intragastric member **500** into the gastric lumen. In an alternative embodiment, the overtube **1000** can include other mechanisms to pull the intragastric member **500** within the lumen of the patient. For example, **FIG. 53** depicts a gear mechanism **1019** comprising a right angled gear component having a plurality of ribs **1021** engaged to the gear mechanism **1019** to facilitate delivery of the intragastric member **500** into the gastric lumen. In another embodiment illustrated in **FIG. 54****,** the overtube **1000** can include a chain **1031** and pulley arrangement to deliver the intragastric member **500** into the gastric lumen, wherein the chain **1031** rotates about and around a crank **1032** to facilitate delivery. Similar to the rollers **1010, 1012,** these alternative devices are mechanically attached to the overtube **1000** or similar delivery device.

Referring to **FIG. 51****,** the intragastric member **500** is loaded into the overtube **1000** by threading an inner delivery tube **1013** through the apertures **512** of each strip **502.** The inner delivery tube **1013** comprises a proximal end 1023 and a distal end **1021** wherein the distal end **1021** comprises a distal tip **1017.** Each of the rollers **1010, 1012** comprise a groove **1018** extending about and around the surface of the roller **1010, 1012** that compliments the shape of the inner delivery tube **1013 (****FIG. 52****).** The grooves **1018** are recessed so as to provide an increase in surface area between the mechanically driven rollers **1010, 1012 (****FIG. 52****)** and the inner delivery tube **1013,** so as to engage the strip **502** therebetween. The rollers **1010, 1012** are mechanically driven and provide tension sufficient to pull the intragastric member 500 along the overtube **1000** during delivery into the gastric lumen. The overtube **1000** further includes a cinching member **1015,** such as nylon thread or similar thread-like structure, which passes through a main lumen of the inner delivery tube **1013** wherein it can be released upon delivery into the gastric lumen.

Delivery of the intragastric member **500** begins by inserting the distal end **1021** of the overtube **1000** in the gastric lumen of the patient while the proximal end **1023** will remain external to the patient. The rollers **1010,1012** of the overtube **1000** facilitate the delivery of each bundle **514** from the overtube **1000** into the gastric lumen. Once the distal end **1021** of the overtube **1000** is positioned in the gastric lumen, the first bundle **514** is threaded over the proximal (rearward) end **1023** of the inner delivery tube **1013** by passing the apertures **512** over the inner delivery tube **1013.** Utilizing the rollers **1010,1012,** each bundle **514** is pulled from the proximal end **1023** of the overtube **1000,** and pulled towards the distal end **1021** of the overtube **1000** so as to pull the folds **504** of the first bundle **514** past the distal end **1021** of the overtube **1000.**

As shown in **FIG. 51****,** the distal tip **1017** of the inner delivery tube **1013** comprises a first diameter **D1,** wherein the remainder of the inner delivery tube **1013** comprises a second diameter **D2.** The first diameter **D1** is sized and configured to facilitate delivery of the intragastric member **500** as it passes across the rollers **1010, 1012.** The second diameter **D2** of the inner delivery tube **1013** is sized and configured to align with the apertures **512** of the strips **502** after each bundle **514** transitions along the length of the overtube **1000** during delivery. As the inner delivery tube **1013** is withdrawn subsequent bundle **514** is pulled past the proximal end **1023** of the inner delivery tube **1013** onto the distal tip **1017.** A skilled artisan would appreciate that other designs comprising an inner delivery tube of different diameters could be utilized.

This procedure is then repeated until all of the bundles **514** have been inserted into the gastric lumen. The bundles **514** are then secured together by pushing a proximal stopper or similar device along the outer delivery tube **518** so as to press against the last bundle **514** to be inserted. The inner delivery tube **1013** is then withdrawn so as to leave the cinching member **510** extending through the apertures **512** of all of the bundles **514.** The cinching member **510** is then tied or otherwise secured to the second stopper **524** so as to form a complete intragastric member **500.**

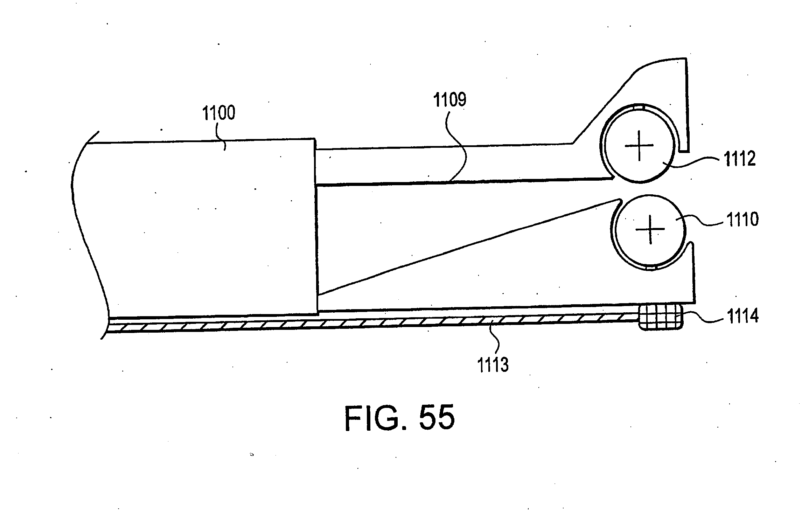

**FIG. 55** depicts another embodiment of a delivery system. In this embodiment, the overtube **1100** comprises rollers **1110,1112** engaged along an inner surface **1109** along a distal end of the overtube **1100.** During delivery of the intragastric member **500,** the rollers **1110, 1112** engage the intragastric member **500** and pull the intragastric member **500** into the gastric lumen of the patient. A flexible shaft **1113** comprising a gearbox **1114** is engaged along the body of the overtube **1100** to support the overtube **1100.** The gearbox **1114** is connected to the rollers **1110, 1112** to regulate the rotation of the rollers **1110,1112** during deliver of the intragastric member **500** into the gastric lumen of the patient.

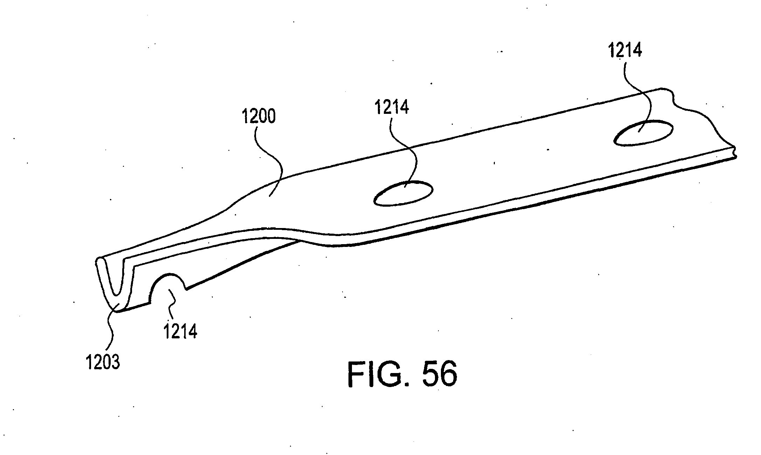

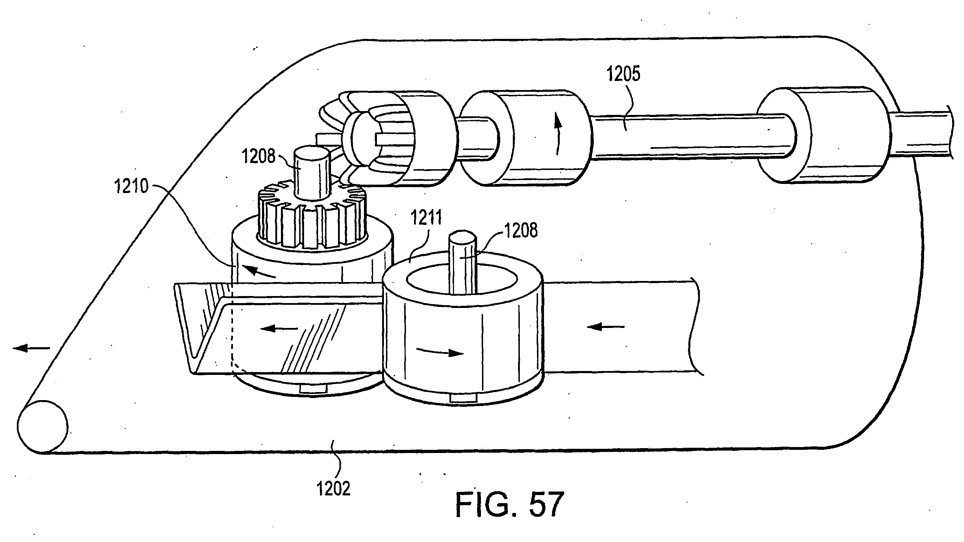

**FIGS. 56-57** depict another embodiment of an intragastric member **1200.** In this embodiment, the intragastric member **1200** comprises a single strip of high-density polyethylene **1202** that comprises a folded edge **1203** along a distal end of the intragastric member **1200.** The intragastric member **1200** further comprises a plurality of apertures **1214** spaced along the intragastric member **1200** that allow the intragastric member **1200** to be threaded onto a delivery mechanism and pulled together upon delivery into the gastric lumen. The folded edge **1203** of the intragastric member **1200** is received by a gear driven roller mechanism **1205.** The gear driven roller mechanism **1205** is attached to an overtube to assist in delivery of the intragastric member **1200.** The gear driven roller mechanism **1205** comprises a pair of rollers **1210,1211** that engage the folded edge **1203** of the intragastric member **1200** and thereby pulls the intragastric member **1200** through the overtube **1202** and into the gastric lumen of the patient. The rollers **1210,1211** are attached to the overtube **1202** with a pair of brackets **1208** engaged to the body of the overtube **1202.** The rollers **1210,1211** can also be attached to utilizing other suitable mechanisms, such as screws and the like, depending on the particular design.

Any other undisclosed or incidental details of the construction or composition of the various elements of the disclosed embodiment of the present invention are not believed to be critical to the achievement of the advantages of the present invention, so long as the elements possess the attributes needed for them to perform as disclosed. The selection of these and other details of construction are believed to be well within the ability of one of even rudimentary skills in this area, in view of the present disclosure. Illustrative embodiments of the present invention have been described in considerable detail for the purpose of disclosing a practical, operative structure whereby the invention may be practiced advantageously. The designs described herein are intended to be exemplary only. The novel characteristics of the invention may be incorporated in other structural forms without departing from the scope of the invention.

## Claims

1. An intragastric device for the treatment of obesity, the intragastric device comprising:
an intragastric member (500,1400) that is re-configurable from a first configuration to a second configuration, the first configuration being sufficiently small to permit introduction of said intragastric member into a gastric lumen of a mammal, the second configuration being sufficiently large to prevent said intragastric device from passing through the mammal's pylorus, the intragastric member comprising a plurality of spaced apart openings;
an outer delivery tube (518, 1718) having a main lumen, a proximal end, and a distal end,
an inner delivery tube (516, 1716) engaged with the main lumen of the outer delivery tube; and
at least one stopper (524, 1729) engaged with the inner delivery tube to secure the intragastric member upon delivery into the gastric lumen,
**characterised in that** the intragastric member is loaded between the proximal end and distal end of the outer delivery tube in the first configuration, the intragastric member being loaded by passing the outer delivery tube through the openings of the intragastric member.

2. The intragastric device according to claim 1 further comprising an overtube having a proximal end, a distal end, and a lumen configured to receive the intragastric member in the first configuration for delivery into the gastric lumen, the outer delivery tube being movably disposed within the lumen of the overtube.

3. The intragastric device according to claim 2 wherein the overtube comprises a plurality of rollers to facilitate delivery of the intragastric member into the gastric lumen.

4. The intragastric device according to claim 1 wherein the inner delivery tube comprises a distal section having a reduced diameter, the reduced diameter being configured to prevent proximal movement of the intragastric member.

5. The intragastric device according to claim 1 wherein the outer delivery tube comprises a flared distal end for preventing proximal movement of the intragastric member.

6. The intragastric device according to claim 1 wherein the intragastric member comprises a continuous strip of material that has been folded to form a plurality of loops, said plurality of loops being connected together to form a shape suggestive of a butterfly or bow-tie in the second configuration.

## Patentansprüche

1. Magensonde zur Behandlung von Adipositas, worin die Magensonde Folgendes umfasst:
ein intragastrisches Element (500, 1400), das von einer ersten Konfiguration in eine zweite Konfiguration umkonfiguriert werden kann, wobei die erste Konfiguration ausreichend klein ist, um die Einführung des intragastrischen Elements in eine Magenlumen eines Säugetiers zu gestatten, die zweite Konfiguration ausreichend groß ist, um zu verhindern, dass die Magensonde durch den Pylorus des Säugetiers gleitet, und das intragastrische Element eine Vielzahl von beabstandeten Öffnungen umfasst;
einen äußeren Zuführungsschlauch (518, 1718) mit einem Hauptlumen, einem proximalen Ende und einem distalen Ende,
einem inneren Zuführungsschlauch (516, 1716), der mit dem Hauptlumen des äußeren Zuführungsschlauchs in Eingriff steht; und
zumindest einen Stopper (524, 1729), der mit dem inneren Zuführungsschlauch in Eingriff steht, um das intragastrische Element nach seiner Einführung in das Magenlumen zu fixieren,
**dadurch gekennzeichnet, dass** das intragastrische Element zwischen dem proximalen Ende und dem distalen Ende des äußeren Zuführungsschlauchs in der ersten Konfiguration geladen wird, wobei das intragastrische Element durch Passieren des äußeren Zuführungsschlauchs durch die Öffnungen des intragastrischen Elements geladen wird.

2. Magensonde nach Anspruch 1, ferner umfassend einen Schutzschlauch mit einem proximalen Ende, einem distalen Ende und einem zur Aufnahme des intragastrischen Elements in der ersten Konfiguration zur Einführung in das Magenlumen ausgelegten Lumen, wobei der äußere Zuführungsschlauch beweglich im Lumen des Schutzschlauchs angeordnet ist.

3. Magensonde nach Anspruch 2, worin der Schutzschlauch einen Vielzahl von Rollen zur Erleichterung der Einführung des intragastrischen Elements in das Magenlumen umfasst.

4. Magensonde nach Anspruch 1, worin der innere Zuführungsschlauch einen distalen Abschnitt mit einem reduzierten Durchmesser umfasst, wobei der reduzierte Durchmesser zur Verhinderung einer proximalen Bewegung des intragastrischen Elements ausgelegt ist.

5. Magensonde nach Anspruch 1, worin der äußere Zuführungsschlauch ein aufgeweitetes distales Ende zur Verhinderung einer proximalen Bewegung des intragastrischen Elements umfasst.

6. Magensonde nach Anspruch 1, worin das intragastrische Element einen kontinuierlichen Materialstreifen umfasst, der zur Bildung einer Vielzahl von Schlaufen gefaltet wurde, wobei die Vielzahl von Schlaufen miteinander verbunden sind, um eine Gestalt zu formen, die in der zweiten Konfiguration den Eindruck eines Schmetterlings oder einer Fliege vermittelt.

## Revendications

1. Dispositif intragastrique destiné au traitement de l'obésité, le dispositif intragastrique comportant :
un élément (500, 1400) intragastrique qui est reconfigurable d'une première configuration à une seconde configuration, la première configuration étant suffisamment petite pour pouvoir introduire ledit élément intragastrique dans une lumière gastrique d'un mammifère, la seconde configuration étant suffisamment grande pour empêcher ledit dispositif intragastrique de traverser le pylore du mammifère, l'élément intragastrique comportant une pluralité d'ouvertures espacées les unes des autres ;
un tube (518, 1718) externe de mise en place présentant une lumière principale, une extrémité proximale, et une extrémité distale,
un tube (516, 1716) interne de mise en place venant en contact avec la lumière principale du tube externe de mise en place ; et
au moins un élément d'arrêt (524, 1729) venant en contact avec le tube interne de mise en place pour fixer l'élément intragastrique lors de la mise en place de ce dernier dans la lumière gastrique,
**caractérisé en ce que** l'élément intragastrique est chargé entre l'extrémité proximale et l'extrémité distale du tube externe de mise en place dans la première configuration, l'élément intragastrique étant chargé en faisant passer le tube externe de mise en place dans les ouvertures de l'élément intragastrique.

2. Dispositif intragastrique selon la revendication 1 comportant en outre un surtube présentant une extrémité proximale, une extrémité distale, et une lumière configurée de manière à recevoir l'élément intragastrique dans la première configuration afin de le mettre en place dans la lumière gastrique, le tube externe de mise en place étant disposé de manière mobile dans la lumière du surtube.

3. Dispositif intragastrique selon la revendication 2 dans lequel le surtube comporte une pluralité de roulettes pour faciliter la mise en place de l'élément intragastrique dans la lumière gastrique.

4. Dispositif intragastrique selon la revendication 1 dans lequel le tube interne de mise en place comporte une section distale d'un diamètre réduit, le diamètre réduit étant configuré de manière à empêcher le déplacement proximal de l'élément intragastrique.

5. Dispositif intragastrique selon la revendication 1 dans lequel le tube externe de mise en place comporte une extrémité distale évasée afin d'empêcher le déplacement proximal de l'élément intragastrique.

6. Dispositif intragastrique selon la revendication 1 dans lequel l'élément intragastrique comporte une bande continue de matériau qui a été pliée afin de créer une pluralité de boucles, les boucles de ladite pluralité de boucles étant reliées ensemble pour créer une forme rappelant celle d'un papillon ou d'un noeud papillon dans la seconde configuration.
